# EUROPÄISCHE PATENTSCHRIFT

(11) **EP 3 122 789 B1**
(45) Veröffentlichungstag und Bekanntmachung des Hinweises auf die Patenterteilung: **08.05.2019**
(21) Anmeldenummer: 15713687.0
(22) Anmeldetag: 26.03.2015
(51) Int. Cl.: C08F 12/30, C08F 12/34, C08F 38/00, H01M 4/60, C08G 65/22, C08G 73/02

(54) **9,10-BIS(1,3-DITHIOL-2-YLIDEN)-9,10-DIHYDROANTHRACENPOLYMERE UND DEREN VERWENDUNG**
9,10-BIS(1,3-DITHIOL-2-YLIDENE)-9,10-DIHYDROANTHRACENE POLYMERS AND USE THEREOF
POLYMÈRES 9,10-BIS(1,3-DITHIOL-2-YLIDÈNE)-9,10-DIHYDROANTHRACÈNE ET LEUR UTILISATION

(30) Priorität: 28.03.2014 DE 102014004760
(43) Veröffentlichungstag der Anmeldung: 01.02.2017
(73) Patentinhaber: Evonik Degussa GmbH, 45128 Essen (DE)
(72) Erfinder: HÄUPLER, Bernhard, 91052 Erlangen (DE); SCHUBERT, Ulrich, 07743 Jena (DE); WILD, Andreas, 99423 Weimar (DE)
(86) Internationale Anmeldenummer: PCT/EP2015/056497
(87) Internationale Veröffentlichungsnummer: WO 2015/144798

(56) Entgegenhaltungen:
- US-A1- 2008 051 463
- MARTA C. DÍAZ ET AL: "Probing Charge Separation in Structurally Different C 60 /exTTF Ensembles", THE JOURNAL OF ORGANIC CHEMISTRY, Bd. 68, Nr. 20, 1. Oktober 2003 (2003-10-01), Seiten 7711-7721, XP055055728, ISSN: 0022-3263, DOI: 10.1021/jo034432e
- Beatriz M. Illescas ET AL: "Supramolecular Threaded Complexes from Fullerene-Crown Ether and [pi]-Extended TTF Derivatives", EUROPEAN JOURNAL OF ORGANIC CHEMISTRY, vol. 2007, no. 30, 1 October 2007 (2007-10-01), pages 5027-5037, XP055391872, DE ISSN: 1434-193X, DOI: 10.1002/ejoc.200700226
- CALBO JOAQUÍN ET AL: "Theoretical insight on novel donor-acceptor exTTF-based dyes for dye-sensitized solar cells", JOURNAL OF MOLECULAR MODELING, SPRINGER BERLIN HEIDELBERG, BERLIN/HEIDELBERG, vol. 20, no. 4, 19 March 2014 (2014-03-19) , pages 1-10, XP035307034, ISSN: 1610-2940, DOI: 10.1007/S00894-014-2188-6 [retrieved on 2014-03-19]
- Tobias Janoschka ET AL: "Abb", , 1 August 2012 (2012-08-01), XP055391885, Retrieved from the Internet: URL:http://onlinelibrary.wiley.com/store/1 0.1002/nadc.201290269/asset/728_ftp.pdf?v= 1&t=j59m1jaz&s=1f357a592fc91c8e8ac857b4f21 efe8d56bac41d [retrieved on 2017-07-18]

## Beschreibung

Die Erfindung betrifft bisher nicht bekannte 9,10-Bis(1,3-dithiol-2-yliden)-9,10-dihydroanthracenpolymere und deren Verwendung als aktive Materialien in elektrischen Ladungsspeichern wie Sekundärbatterien. In solchen Sekundärbatterien können die erfindungsgemäßen Polymere beispielsweise als aktives Elektrodenmaterial eingesetzt werden. Diese Sekundärbatterien zeichnen sich insbesondere durch hohe Zellspannungen, große Leistungsdichten sowie lange Lebensdauer sowie einfache und skalierbare Verarbeitungs- und Herstellungsmethoden aus.
Die neue 9,10-Bis(1,3-dithiol-2-yliden)-9,10-dihydroanthracen (exTTF) Strukturen dieser neuen Polymere zeigen ein besonderes elektrochemisches Verhalten. Dieses zeichnet sich durch einen reversiblen Zwei-Elektronen-Redoxprozess aus, durch welchen die elektrischen Ladungsspeicher unter anderem ein einstufiges Lade/Entladeplateau aufweisen.

Homo- und Copolymere mit anhängenden 9,10-Bis(1,3-dithiol-2-yliden)-9,10-dihydro-anthraceneinheiten und deren Derivaten sind der Fachwelt nicht bekannt.

Organische Radikalbatterien sind elektrochemische Zellen, die ein organisches Ladungsspeicherungsmaterial als aktives Elektrodenmaterial zur Speicherung von elektrischer Ladung verwenden. Diese Sekundärbatterien zeichnen sich durch ihre besonderen Eigenschaften, wie Schnellladefähigkeit, hohe Lebensdauer, geringes Gewicht und hohe Flexibilität und einfach Verarbeitbarkeit, aus. Einige polymere Strukturen mit anderen redoxaktiven Einheiten als die vorgenannten neuen 9,10-Bis(1,3-dithiol-2-yliden)-9,10-dihydroanthracenpolymere sind hingegen als aktive Elektrodenmaterialien zur Ladungsspeicherung bereits bekannt (beispielsweise zeigen die WO 2012133202 A1, WO 2012133204 A1, WO 2012120929 A1, WO 2012153866 A1, WO 2012153865 A1, JP 2012-221574 A, JP 2012-221575 A, JP 2012-219109 A, JP 2012-079639 A, WO 2012029556 A1, WO 2012153865 A1, JP 2011-252106 A, JP 2011-074317 A, JP 2011-165433 A, WO 2011034117 A1, WO 2010140512 A1, WO 2010104002 A1, JP 2010-238403 A, JP 2010-163551 A, JP 2010-114042 A, WO 2010002002 A1, WO 2009038125 A1, JP 2009-298873 A, WO 2004077593 A1, WO 2009145225 A1, JP 2009-238612 A, JP 2009-230951 A, JP 2009-205918 A, JP 2008-234909 A, JP 2008-218326 A, WO 2008099557 A1, WO 2007141913 A1, US 20020041995 A1, US 20020041995 A1, JP 2002-117852 A, EP 1128453 A2, polymere Verbindungen mit organischen Nitroxidradikalen als aktive Einheiten zur Ladungsspeicherung); US 20020041995, JP 2002-117852 A zeigen als Beispiel polymere Verbindungen mit organischen Phenoxylradikalen oder Galvinoxylradikalen).

Auch polymere Verbindungen mit Chinonen (beispielsweise JP 2009-217992 A, WO 2013/099567 A1, WO 2011/068217 A1), mit Dionen (beispielsweise JP 2010-212152 A), sowie mit Dicyanodiiminen (beispielsweise JP 2012-190545 A, JP 2010-55923 A) als aktive Einheiten zur Ladungsspeicherungen sind bekannt.
Janoschka et al., Nachrichten aus der Chemie 2012, 60, 728 - 731 geben eine Übersicht über konjugierte leitfähige Polymere [z.B. Poly(acetylen), Poly(anilin), Poly(pyrrol)] und Redoxpolymere wie Anthrachinone, Thioether, Disulfide und Nitroxidradikale. Dieser Artikel diskutiert auch den Einsatz dieser Materialien in organischen, polymerbasierten Batterien.
Wie beschrieben, sind der Fachwelt keine erfindungsgemäßen 9,10-Bis(1,3-dithiol-2-yliden)-9,10-dihydroanthracenpolymere bekannt und finden deshalb keine Verwendung als elektrische Ladungsspeicher.

Die theoretische Kapazität der bereits bekannten Polymere mit redoxaktiven Substituenten ist durch zwei Faktoren streng limitiert. Einerseits durch die molare Masse der Monomereinheit und andererseits durch die Anzahl an Elektronen, die an der Redoxreaktion der elektrischen Ladungsspeicherung beteiligt sind. Die meisten organischen redoxaktiven Einheiten verfügen nur über einen Ein-Elektronenprozess, welcher zur Ladungsspeicherung verwendet wird, und weisen deshalb, wie oben angemerkt, auf Grund ihrer vergleichsweise hohen Molmasse eine niedrige theoretische Kapazität auf.
Durch die Verwendung von Mehrelektronredoxprozessen, wie dies z. B. bei Chinonen oder Dicyanodiimiden der Fall ist, erhöht sich die theoretische Kapazität des Materials, jedoch sind diese Mehrelektronenprozesse abhängig von einander, so dass die Redoxreaktionen bei unterschiedlichen Potentialen stattfinden und somit mehrere unerwünschte Lade-/Entladeplateaus bei unterschiedlichen Zellspannungen im jeweiligen elektrischen Ladungsspeicher entstehen. Des weiteren werden Chinone und Dicyanodiimide in der jeweiligen Redoxreaktion reduziert und weisen somit ein niedriges Redoxpotential auf, welches im elektrischen Ladungsspeicher zu einer geringen Zellspannung führt.

Der Erfindung liegt deshalb der Aufgabe zugrunde, neue Polymere zu schaffen, die mit geringem Aufwand herstellbar sind, wobei deren chemisch-physikalischen Eigenschaften bei der Synthese in weiten Grenzen gezielt beeinflusst werden können, und welche als aktive Materialien in elektrischen Ladungsspeichern mit hoher Zellspannung für hohe Speicherkapazität, lange Lebensdauer und ein flaches Lade-/Entladeplateau und verwendet werden können.

Es wurden neue 9,10-Bis(1,3-dithiol-2-yliden)-9,10-dihydroanthracenpolymere, bestehend aus einer oligomeren oder polymeren Verbindung der allgemeinen Formel (I) gefunden: 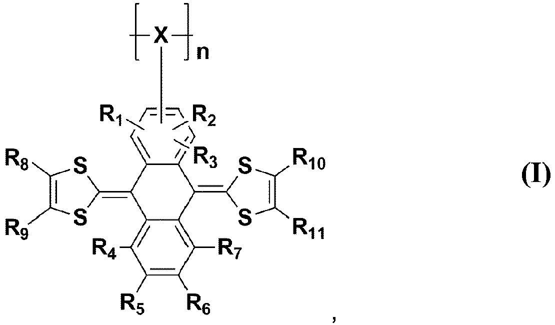 wobei
- R₁ bis R₇:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens fünf der Substituenten R₁ bis R₇ und Nicht-Wasserstoffatome, vorzugsweise Halogenatome, Alkylgruppen, Alkoxygruppen, Cyanogruppen und/oder Nitrogruppen, als null bis zwei der Substituenten R₁ bis R₇ besonders bevorzugt sind,
- R₈ bis R₁₁:: unabhängig voneinander Wasserstoffatome, Alkylgruppen, Alkenylgruppen, Alkinylgruppen, Alkoxygruppen, Alkylthiogruppen, Halo alkylgruppen, Haloalkoxygruppen, Cycloalkylgruppen, Cycloalkoxygruppen, Arylgruppen, Heteroarylgruppen, Aryloxygruppen, Aralkylgruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen können, wobei die Substituenten R₈ und R₉ bzw. die Substituenten R₁₀ und R₁₁ einen weiteren Ring bilden können, der aus fünf bis sieben Atomen besteht (der Ring kann aromatisch, heteroaromatisch, oder nicht aromatisch sein; ist der Ring nicht aromatisch, so kann er aus verschiedenen Gruppen, wie beispielsweise Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminoruppen, Monoalkylaminogruppen, Dialkylaminogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, bestehen, besonders bevorzugt entsprechen sich R₈ bis R₁₁ und sind Alkylgruppen, wie typischerweise Methylgruppen oder Ethylgruppen, Alkylthiogruppen, wie Methylthiogruppen, oder Ethylthiogruppen und Thiolgruppen),
- X:: eine organische Gruppe ist, welche durch Polymerisationreaktion aus einer Gruppe gebildet wird, die aus einer organischen Doppelbindung, einer organischen Dreifachbindung, eines Oxirans oder eines Aziridins besteht, oder eine organische Gruppe ist, welche durch eine polymeranaloge Reaktion gebildet wird,
- n:: eine Ganzzahl größer gleich 2 ist.
Die organische Gruppe X kann dabei vorzugsweise eine Struktur der nachstehenden Formeln II - XIV aufweisen: 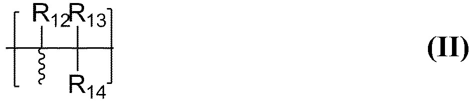 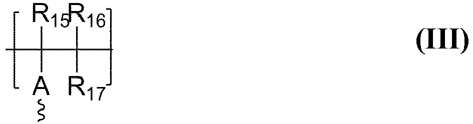 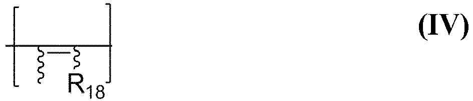 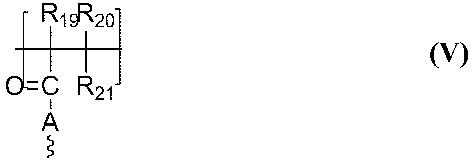 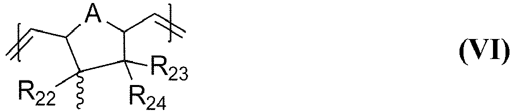 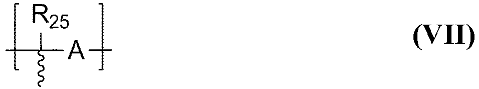 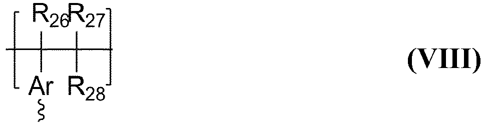 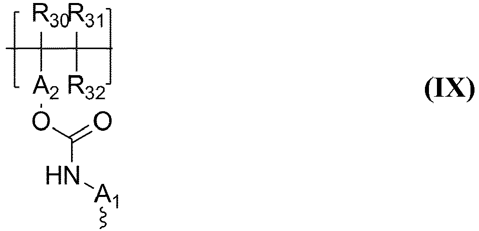 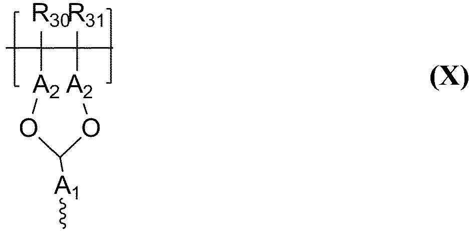 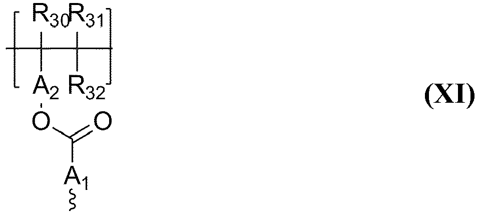 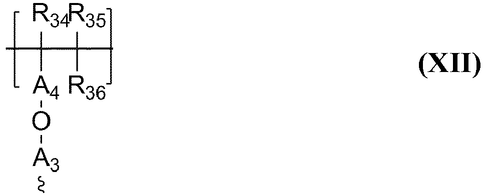 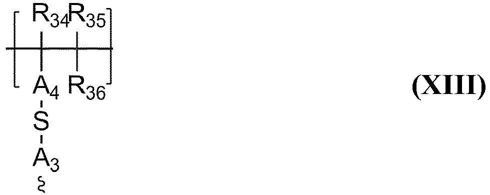 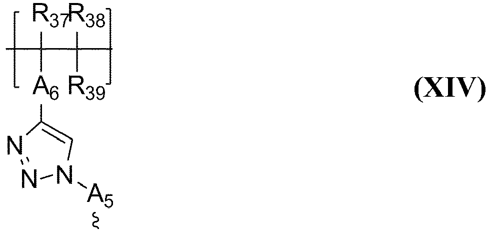 wobei
- R₁₂ bis R₂₈:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei
Wasserstoffatome als mindestens zwei der Substituenten R₁₂ bis R₁₄ und Nicht-Wasserstoffatome, vorzugsweise Halogenatome, Alkylgruppen, Alkoxygruppen, Cyanogruppen und/oder Nitrogruppen, als null bis zwei der Substituenten R₁₂ bis R₁₄ und/oder
Wasserstoffatome als mindestens zwei der Substituenten R₁₅ bis R₁₇ und Nicht-Wasserstoffatome, vorzugsweise Halogenatome, Alkylgruppen, Alkoxygruppen, Cyanogruppen und/oder Nitrogruppen, als null bis einem der Substituenten R₁₅ bis R₁₇ und/oder
ein Wasserstoffatom als R₁₈, und/oder
Wasserstoffatome als mindestens zwei der Substituenten R₁₉ bis R₂₁ und Nicht-Wasserstoffatome, vorzugsweise Halogenatome, Alkylgruppen, Alkoxygruppen, Cyanogruppen und/oder Nitrogruppen, als null bis einem der Substituenten R₁₉ bis R₂₁, und/oder
Wasserstoffatome als mindestens zwei der Substituenten R₂₂ bis R₂₄ und Nicht-Wasserstoffatome, vorzugsweise Halogenatome, Alkylgruppen, Alkoxygruppen, Cyanogruppen und/oder Nitrogruppen, als null bis einem der Substituenten R₂₂ bis R₂₄ und/oder
ein Wasserstoffatom als R₂₅ und/oder
Wasserstoffatome als mindestens zwei der Substituenten R₂₆ bis R₂₈ und Nicht-Wasserstoffatome, vorzugsweise Halogenatome, Alkylgruppen, Alkoxygruppen, Cyanogruppen und/oder
Nitrogruppen, als null bis einem der Substituenten R₂₆ bis R₂₈
besonders bevorzugt sind,
- R₃₀ bis R₃₂:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens zwei der Substituenten R₃₀ bis R₃₂ und Nicht-Wasserstoffatome, vorzugsweise Halogenatome, Alkylgruppen, Alkoxygruppen, Cyanogruppen und/oder Nitrogruppen, als null bis einem der Substituenten R₃₀ bis R₃₂ besonders bevorzugt sind,
- R₃₄ bis R₃₆:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Halogenatome oder eine Kombination dieser Gruppen oder Atome darstellen, wobei Wasserstoffatome als mindestens zwei der Substituenten R₃₄ bis R₃₆, und Nicht-Wasserstoffatome, vorzugsweise Halogenatome, Alkylgruppen, Alkoxygruppen, Cyanogruppen und/oder Nitrogruppen, als null bis einem der Substituenten R₃₄ bis R₃₆ besonders bevorzugt sind,
- R₃₇ bis R₃₉:: unabhängig voneinander Wasserstoffatome, Alkylgruppen, Alkenylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkyl
aminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens zwei der Substituenten R₃₇ bis R₃₉ und Nicht-Wasserstoffatome, vorzugsweise Halogenatome, Alkylgruppen, Alkoxygruppen, Cyanogruppen und/oder Nitrogruppen, als null bis einem der Substituenten R₃₇ bis R₃₉ besonders bevorzugt sind,
- A:: ein Sauerstoffatom, ein Schwefelatom oder eine -N(R₃₃)- Gruppe ist, wobei R₃₃ ein Wasserstoffatom, eine Alkylgruppe, eine Alkenylgruppe, eine Alkinylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Carbonsäuregruppe, eine Sulfonsäuregruppe, eine Nitrogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe ist, wobei ein Sauerstoffatom als A besonders bevorzugt ist,
- A₁ und A₂:: unabhängig voneinander eine kovalente Bindung, eine Alkylgruppe, eine Alkenylgruppe, eine Alkinylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung oder eine Alkylgruppe als A₁ und A₂ besonders bevorzugt ist,
- A₃ und A₄:: unabhängig voneinander eine kovalente Bindung, eine Alkylgruppe, eine Alkenylgruppe, eine Alkinylgruppe, eine Alkoxygruppe, eine Alkylthiogruppe, eine Cycloalkylgruppe, eine Cycloalkoxygruppe, eine Arylgruppe, eine Heteroarylgruppe, eine Aryloxygruppe, eine Aralkylgruppe,
eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung oder eine Alkylgruppe als A₃ und A₄ besonders bevorzugt ist,
- A₅ und A₆:: unabhängig voneinander eine kovalente Bindung, eine Alkylgruppe, eine Alkenylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung, eine Arylgruppe oder eine Alkylgruppe als A₅ und A₆ besonders bevorzugt ist,
- Ar:: einer unabhängig voneinander substituierten Cycloalkylgruppe, Cycloalkoxygruppe, Arylgruppe, Heteroarylgruppe, Aryloxygruppe, Aralkylgruppe entspricht.

Es wurden neue Polymere synthetisiert, welche 9,10-Bis(1,3-dithiol-2-yliden)-9,10-dihydroanthraceneinheiten als anhängende Gruppe am Polymerrückgrat enthalten. Diese Polymere weisen hervorragende Eigenschaften, insbesondere als redoxaktives Elektrodenmaterial in Kathoden für sekundäre elektrische Ladungsspeicher, auf. In den Unteransprüchen sind vorteilhafte Verwendungsmöglichkeiten der erfindungsgemäßen 9,10-Bis(1,3-dithiol-2-yliden)-9,10-dihydroanthracenpolymere genannt.

Diese neuen Polymere können auf einfache und aufwandgeringe Weise sowie aus gut zugänglichen Startmaterialien hergestellt werden. Zur Polymerisation ist kein weiteres Monomer nötig und die Polymerisation benötigt keine teuren Metallkatalysatoren, sondern als Herstellungsmethode können einfache Polymerisationsverfahren verwendet werden. Dabei können Polymere mit hoher Molmasse und geringem Polydispersitätsindex in sehr hohen Ausbeuten erhalten werden. Durch die Einführung von polymerisierbaren Gruppen mit geringer Molmasse kann die Monomermolmasse niedrig gehalten und die theoretische Kapazität des sekundären elektrischen Ladungsspeichers maximiert werden. Ferner stehen in diesen Polymeren die redoxaktiven Gruppen nicht in Konjugation miteinander; als Konsequenz weist der elektrische Ladungsspeicher ein flaches Lade-/Entladeplateau auf. Diese Materialien unterscheiden sich vom Stand der Technik durch eine Zwei-Elektroden-Redoxreaktion, welche zu dem besagten flachen Lade-/Entladeplateau bei gleichzeitiger hoher Kapazität und hoher Lebensdauer im Bauteil führt.

In der folgenden Beschreibung definiert sich n als normal, i als iso, s als sekundär, t als tertiär, c als cyclo, m als meta, p als para und o als ortho.
In dieser Spezifikation kann eine Alkylgruppe sowohl verzweigt als auch unverzweigt sein. Eine Alkylgruppe besteht typischerweise aus einem bis zu dreißig Kohlenstoffatomen, bevorzugt aus einem bis zu zwanzig Kohlenstoffatomen. Beispiele für eine Alkylgruppe sind: Methylgruppe, Ethylgruppe, Propylgruppe, Isopropylgruppe, n-Butylgruppe, sec-Butylgruppe, t-Butylgruppe, Pentylgruppe, n-Hexylgruppe, n-Heptylgruppe, 2-Ethylhexylgruppe, n-Octylgruppe, n-Nonylgruppe, n-Decylgruppe, n-Undecylgruppe, n-Dodecylgruppe, n-Tridecylgruppe, n-Tetradecylgruppe, n-Pentadecylgruppe, n-Hexadecylgruppe, n-Heptadecylgruppe, n-Octadecylgruppe, n-Nonadecylgruppe oder Eicosylgruppe. Besonders bevorzugt sind Alkylgruppen mit einem bis sechs Kohlenstoffatomen.

In dieser Spezifikation kann eine Alkenylgruppe sowohl verzweigt als auch unverzweigt sein. Eine Alkenylgruppe besteht typischerweise aus einem bis zu dreißig Kohlenstoffatomen, bevorzugt aus einem bis zwanzig Kohlenstoffatomen. Alkenylgruppen besitzen typischerweise eine ungesättigte ethenylsiche Doppelbindung, der restliche Anteil der Alkenylgruppe ist gesättigt. Zwei oder mehrere ethenylische ungesättigte Doppelbindungen sind möglich, aber nicht bevorzugt. Besonders bevorzugt ist die ungesättigte ethenylische Doppelbindung an der alpha Position der Alkenylgruppe. Beispiele für eine Alkenylgruppe sind: Vinylgruppe, Allylgruppe, Propenylgruppe, Isopropenylgruppe, n-Butenylgruppe, sec.-Butenylgruppe, Pentenylgruppe, n-Hexenylgruppe, n-Heptenylgruppe, 2-Ethylhexenylgruppe, n-Octenylgruppe, n-Nonenylgruppe, n-Decenylgruppe, n-Undecenylgruppe, n-Dodecenylgruppe, n-Tridecenylgruppe, n-Tetradecenylgruppe, n-Pentadecenylgruppe, n-Hexadecenylgruppe, n-Heptadecenylgruppe, n-Octadecenylgruppe, n-Nonadecenylgruppe oder Eicosenylgruppe. Bevorzugt sind Alkenylgruppen mit zwei bis drei Kohlenstoffatomen; besonderes bevorzugt sind Vinylgruppen und Allylgruppen.

In dieser Spezifikation kann eine Alkinylgruppe sowohl verzweigt als auch unverzweigt sein. Eine Alkinylgruppe besteht typischerweise aus zwei bis zu dreißig Kohlenstoffatomen, bevorzugt aus einem bis zwanzig Kohlenstoffatomen. Alkinylgruppen besitzen typischerweise eine ungesättigte ethinylische Dreifachbindung, der restliche Anteil der Alkinylgruppe ist gesättigt. Zwei oder mehrere ethinylische ungesättigte Dreifachbindungen sind möglich, aber nicht bevorzugt. Besonders bevorzugt ist die ungesättigte ethinylische Doppelbindung an der alpha-Position der Alkinylgruppe. Beispiele für eine Alkinylgruppe sind: Ethinylgruppe, Propinylgruppe, Butinylgruppe, Pentinylgruppe, n-Hexinylgruppe, n-Heptinylgruppe, 2-Ethylhexinylgruppe, n-Octyinlgruppe, n-Noninylgruppe, n-Decinylgruppe, n-Undecinylgruppe, n-Dodecinylgruppe, n-Tridecinylgruppe, n-Tetradecinylgruppe, n-Pentadecinylgruppe, n-Hexadecinylgruppe, n-Heptadecinylgruppe, n-Octadecinylgruppe, n-Nonadecinylgruppe oder Eicosinylgruppe. Bevorzugt sind Alkinylgruppen mit zwei Kohlenstoffatomen.

In dieser Spezifikation kann eine Alkylthiogruppe sowohl verzweigt als auch unverzweigt sein. Eine Alkylthiogruppe besteht typischerweise aus einem bis zu dreißig Kohlenstoffatomen und einem oder mehreren Schwefelatomen, die kovalent an zwei Kohlenstoffatomen der Kette gebunden sind, bevorzugt aus einem bis zwanzig Kohlenstoffatomen und einem Schwefelatom. Beispiele für eine Alkylthiogruppe sind: Methylthiogruppe, Ethylthiogruppe, n-Propylthiogruppe, i-Propylthiogruppe, n-Butylthiogruppe, s-Butylthiogruppe, t-Butylthiogruppe, n-Pentylthiogruppe, 1-Methylbutylthiogruppe, 2-Methylbutylthiogruppe, 3-Methylbutylthiogruppe, 1,1-Dimethylpropylthiogruppe, 2,2-Dimethylpropylthiogruppe, n-Hexylthiogruppe, 1-Methylpentylthiogruppe, 2-Methylpentylthiogruppe, 1,1-Dimethylbutylthiogruppe, 1-Ethylbutylthiogruppe, 1,1,2-Trimethylpropylthiogruppe, n-Heptylthiogruppe, n-Octylthiogruppe, 2-Ethylhexylthiogruppe, n-Nonylthiogruppe, n-Decylthiogruppe, n-Dodecylthiogruppe.

In dieser Spezifikation kann eine Monoalkylaminogruppe sowohl verzweigt als auch unverzweigt sein. Eine Monoalkylaminogruppe besteht typischerweise aus einem bis zu dreißig Kohlenstoffatomen und einem oder mehreren Stickstoffatomen, die kovalent an zwei Kohlenstoffatomen der Kette gebunden sind, bevorzugt aus einem bis zwanzig Kohlenstoffatomen und einem Stickstoffatom. Beispiele für eine Monoalkylaminogruppe sind: Methylaminogruppe, Ethylaminogruppe, n-Propylaminogruppe, i-Propylaminogruppe, c-Propylaminogruppe, n-Butylaminogruppe, i-Butylaminogruppe, s-Butylaminogruppe, t-Butylaminogruppe, c-Butylaminogruppe, 1-Methyl-c-propylaminogruppe, 2-Methyl-c-propylaminogruppe, n-Pentylaminogruppe, 1-Methyl-n-butylaminogruppe, 2-Methyl-n-butylaminogruppe, 3-Methyl-n-butylaminogruppe, 1,1-Dimethyl-n-propylaminogruppe, 1,2-Dimethyl-n-propylaminogruppe, 2,2-Dimethyl-n-propylaminogruppe, 1-Ethyl-n-propylaminogruppe, c-Pentylaminogruppe, 1-Methyl-c-butylaminogruppe, 2-Methyl-c-butylaminogruppe, 3-Methyl-c-butylaminogruppe, 1,2-Dimethyl-c-propylaminogruppe, 2,3-Dimethyl-c-propylaminogruppe, 1-Ethyl-c-propylaminogruppe, 2-Ethyl-c-propylaminogruppe, n-Hexylaminogruppe, 1-Methyl-n-pentylaminogruppe, 2-Methyl-n-pentylaminogruppe, 3-Methyl-n-pentylaminogruppe, 4-Methyl-n-pentylaminogruppe, 1,1-Dimethyl-n-butylaminogruppe, 1,2-Dimethyl-n-butylaminogruppe, 1,3-Dimethyl-n-butylaminogruppe, 2,2-Dimethyl-n-butylaminogruppe, 2,3-Dimethyl-n-butylaminogruppe, 3,3-Dimethyl-n-butylaminogruppe, 1-Ethyl-n-butylaminogruppe, 2-Ethyl-n-butylaminogruppe, 1,1,2-Trimethyl-n-propylaminogruppe, 1,2,2-Trimethyl-n-propylaminogruppe, 1-Ethyl-1-methyl-n-propylaminogruppe, 1-Ethyl-2-methyl-n-propylaminogruppe, c-Hexylaminogruppe, 1-Methyl-c-pentylaminogruppe, 2-Methyl-c-pentylaminogruppe, 3-Methyl-c-pentylaminogruppe, 1-Ethyl-c-butylaminogruppe, 2-Ethyl-c-butylaminogruppe, 3-Ethyl-c-butylaminogruppe, 1,2-Dimethyl-c-butylaminogruppe, 1,3-Dimethyl-c-butylaminogruppe, 2,2-Dimethyl-c-butylaminogruppe, 2,3-Dimethyl-c-butylaminogruppe, 2,4-Dimethyl-c-butylaminogruppe, 3,3-Dimethyl-c-butylaminogruppe, 1-n-Propyl-c-propylaminogruppe, 2-n-Propyl-c-Propylaminogruppe, 1-i-Propyl-c-Propylaminogruppe, 2-i-Propyl-c-propylaminogruppe, 1,2,2-Trimethyl-c-propylaminogruppe, 1,2,3-Trimethyl-c-propylaminogruppe, 2,2,3-Trimethyl-c-propylaminogruppe, 1-Ethyl-2-methyl-c-propylaminogruppe, 2-Ethyl-1-methyl-c-propylaminogruppe, 2-Ethyl-2-methyl-c-propylaminogruppe, 2-Ethyl-3-methyl-c-propylaminogruppe.

In dieser Spezifikation kann eine Dialkylaminogruppe sowohl verzweigt als auch unverzweigt sein. Eine Dialkylaminogruppe besteht typischerweise aus einem bis zu dreißig Kohlenstoffatomen und einem oder mehreren Stickstoffatomen, die kovalent an drei Kohlenstoffatomen der Kette gebunden sind, bevorzugt aus einem bis zwanzig Kohlenstoffatomen und einem Stickstoffatom. Beispiele für eine Dialkylaminogruppe sind: Di-i-propylaminogruppe, Di-c-propylaminogruppe, Di-n-butylaminogruppe, Di-i-butylaminogruppe, Di-s-butylaminogruppe, Di-t-butylaminogruppe, Di-c-butylaminogruppe, Di-(1-methyl-c-propyl)aminogruppe, Di-(2-methyl-c-propyl)aminogruppe, Di-n-pentylaminogruppe, Di-(1-methyl-n-butyl)aminogruppe, Di-(2-methyl-n-butyl)aminogruppe, Di-(3-methyl-n-butyl)aminogruppe, Di-(1,1-dimethyl-n-propyl)aminogruppe, Di-(1,2-dimethyl-n-propyl)aminogruppe, Di-(2,2-dimethyl-n-propyl)aminogruppe, Di-(1-ethyl-n-propyl)aminogruppe, Di-c-pentylaminogruppe, Di-(1-methyl-c-butyl)aminogruppe, Di-(2-methyl-c-butyl)aminogruppe, Di-(3-methyl-c-butyl)aminogruppe, Di-(1,2-dimethyl-c-propyl)aminogruppe, Di-(2,3-dimethyl-c-propyl)aminogruppe, Di-(1-ethyl-c-propyl) aminogruppe, Di-(2-ethyl-c-propyl)aminogruppe, Di-n-hexylaminogruppe, Di-(1-methyl-n-pentyl)aminogruppe, Di-(2-methyl-n-pentyl)aminogruppe, Di-(3-methyl-n-pentyl)amino gruppe, Di-(4-methyl-n-pentyl)aminogruppe, Di-(1,1-dimethyl-n-butyl)aminogruppe, Di-(1,2-dimethyl-n-butyl)aminogruppe, Di-(1,3-dimethyl-n-butyl)aminogruppe.

In dieser Spezifikation kann eine Haloalkylgruppe sowohl verzweigt als auch unverzweigt sein. Eine Haloalkylgruppe besteht typischerweise aus einem bis zu dreißig Kohlenstoffatomen, die wiederum unabhängig voneinander mit einem oder mehreren Halogenatomen substituiert sein können, bevorzugt aus einem bis zwanzig Kohlenstoffatomen. Beispiele für Halogenatome sind Fluoratom, Chloratom, Bromatom und Iodatom. Bevorzugt sind das Fluoratom und das Chloratom. Beispiele für eine Haloalkylgruppe sind: Difluoromethoxygruppe, Trifluoromethoxygruppe, Bromodifluoromethoxygruppe, 2-Chloroethoxygruppe, 2-Bromoethoxygruppe, 1,1-Difluoroethoxygruppe, 2,2,2-Trifluoroethoxygruppe, 1,1,2,2-Tetrafluoroethoxygruppe, 2-Chloro-1,1,2-trifluoroethoxygruppe, Pentafluoroethoxygruppe, 3-Bromopropoxygruppe, 2,2,3,3-Tetrafluoropropoxygruppe, 1,1,2,3,3,3-Hexafluoropropoxygruppe, 1,1,1,3,3,3-Hexafluoropropoxygruppe, 3-Bromo-2-methylpropoxygruppe, 4-Bromobutoxygruppe, Perfluoropentyloxygruppe.

In dieser Spezifikation kann eine Haloalkoxygruppe sowohl verzweigt als auch unverzweigt sein. Eine Haloalkoxygruppe besteht typischerweise aus einem Sauerstoffatom, an dem eine Kette, bestehend aus einem bis zu dreißig Kohlenstoffatomen, kovalent gebunden ist und die sowohl verzweigt, als auch unverzweigt sein kann sowie deren Kohlenstoffatome wiederum unabhängig voneinander mit einem oder mehreren Halogenatomen substituiert sein können. Bevorzugt besteht diese Kette aus einem bis zwanzig Kohlenstoffatomen. Beispiele für Halogenatome sind Fluoratom, Chloratom, Bromatom und Iodatom. Bevorzugt sind das Fluoratom und das Chloratom. Beispiele für eine Haloalkoxylgruppe sind: Difluoromethoxylgruppe, Trifluoromethoxylgruppe, Bromodifluoromethoxylgruppe, 2-Chloroethoxylgruppe, 2-Bromoethoxylgruppe, 1,1-Difluoroethoxylgruppe, 2,2,2-Trifluoroethoxylgruppe, 1,1,2,2-Tetrafluoroethoxylgruppe, 2-Chloro-1,1,2-trifluoroethoxylgruppe, Pentafluoroethoxylgruppe, 3-Bromopropoxylgruppe, 2,2,3,3-Tetrafluoropropoxylgruppe, 1,1,2,3,3,3-Hexafluoropropoxylgruppe, 1,1,1,3,3,3-Hexafluoropropoxylgruppe, 3-Bromo-2-methylpropoxylgruppe, 4-Bromobutoxylgruppe, Perfluoropentoxylgruppe.

Eine Alkylcarbonylgruppe besteht in dieser Spezifikation typischerweise aus einem Karbonylkohlenstoff, an dem eine Alkylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkylkarbonylgruppe sind: Methylcarbonylgruppe, Ethylcarbonylgruppe, n-Propylcarbonylgruppe, i-Propylcarbonylgruppe, c-Propylcarbonylgruppe, n-Butylcarbonylgruppe, i-Butylcarbonylgruppe, s-Butylcarbonylgruppe, t-Butylcarbonylgruppe, c-Butylcarbonylgruppe, 1-Methyl-c-propylcarbonylgruppe, 2-Methyl-c-propylcarbonylgruppe, n-Pentylcarbonylgruppe, 1-Methyl-n-butylcarbonylgruppe, 2-Methyl-n-butylcarbonylgruppe, 3-Methyl-n-butylcarbonylgruppe, 1,1-Dimethyl-n-propylcarbonylgruppe, 1,2-Dimethyl-n-propylcarbonylgruppe, 2,2-Dimethyl-n-propylcarbonylgruppe, 1-Ethyl-n-propylcarbonylgruppe, c-Pentylcarbonylgruppe, 1-Methyl-c-butylcarbonylgruppe, 2-Methyl-c-butylcarbonylgruppe, 3-Methyl-c-butylcarbonylgruppe, 1,2-Dimethyl-c-propylcarbonylgruppe, 2,3-Dimethyl-c-propylcarbonylgruppe, 1-Ethyl-c-propylcarbonylgruppe, 2-Ethyl-c-propylcarbonylgruppe, n-Hexylcarbonylgruppe. 1-Methyl-n-pentylcarbonylgruppe, 2-Methyl-n-pentylcarbonylgruppe, 3-Methyl-n-pentylcarbonylgruppe, 4-Methyl-n-pentylcarbonylgruppe, 1,1-Dimethyl-n-butylcarbonylgruppe, 1,2-Dimethyl-n-butylcarbonylgruppe, 1,3-Dimethyl-n-butylcarbonylgruppe, 2,2-Dimethyl-n-butylcarbonylgruppe, 2,3-Dimethyl-n-butylcarbonylgruppe, 3,3-Dimethyl-n-butylcarbonylgruppe, 1-Ethyl-n-butylcarbonylgruppe, 2-Ethyl-n-butylcarbonylgruppe.

Eine Alkenylcarbonylgruppe in dieser Spezifikation besteht typischerweise aus einem Karbonylkohlenstoff, an dem eine Alkenylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkenylkarbonylgruppe sind: Ethenylcarbonylgruppe, 1-Propenylcarbonylgruppe, 2-Propenylcarbonylgruppe, 1-Methyl-1-ethenylcarbonylgruppe, 1-Butenylcarbonylgruppe, 2-Butenylcarbonylgruppe, 3-Butenylcarbonylgruppe, 2-Methyl-1-propenylcarbonylgruppe, 2-Methyl-2-propenylcarbonylgruppe, 1-Ethylethenylcarbonylgruppe, 1-Methyl-1-propenylcarbonylgruppe, 1-Methyl-2-propenylcarbonylgruppe, 1-Pentenylcarbonylgruppe, 2-Pentenylcarbonylgruppe, 3-Pentenylcarbonylgruppe, 4-Pentenylcarbonylgruppe, 1-n-Propylethenylcarbonylgruppe, 1-Methyl-1-butenylcarbonylgruppe, 1-Methyl-2-butenylcarbonylgruppe, 1-Methyl-3-butenylcarbonylgruppe, 2-Ethyl-2-propenylcarbonylgruppe, 2-Methyl-1-butenylcarbonylgruppe, 2-Methyl-2-butenylcarbonylgruppe, 2-Methyl-3-butenylcarbonylgruppe, 3-Methyl-1-butenylcarbonylgruppe, 3-Methyl-2-butenylcarbonylgruppe, 3-Methyl-3-butenylcarbonylgruppe, 1,1-Dimethyl-2-propenylcarbonylgruppe, 1-i-Propylethenylcarbonylgruppe, 1,2-Dimethyl-1-propenylcarbonylgruppe, 1,2-Dimethyl-2-propenylcarbonylgruppe, 1-c-Pentenylcarbonylgruppe, 2-c-Pentenylcarbonylgruppe, 3-c-Pentenylcarbonylgruppe, 1-Hexenylcarbonylgruppe, 2-Hexenylcarbonylgruppe, 3-Hexenylcarbonylgruppe, 4-Hexenylcarbonylgruppe, 5-Hexenylcarbonylgruppe, 1-Methyl-1-pentenylcarbonylgruppe, 1-Methyl-2-pentenylcarbonylgruppe, 1-Methyl-3-pentenylcarbonylgruppe, 1-Methyl-4-Pentenylcarbonylgruppe, 1-n-Butylethenylcarbonylgruppe, 2-Methyl-1-pentenylcarbonylgruppe, 2-Methyl-2-pentenylcarbonylgruppe, 2-Methyl-3-pentenylcarbonylgruppe, 2-Methyl-4-pentenylcarbonylgruppe, 2-n-Propyl-2-propenylcarbonylgruppe, 3-Methyl-1-pentenylcarbonylgruppe, 3-Methyl-2-pentenylcarbonylgruppe.

Eine Alkinylcarbonylgruppe besteht in dieser Spezifikation typischerweise aus einem Karbonylkohlenstoff, an dem eine Alkinylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkinylkarbonylgruppe sind: Ethinylcarbonylgruppe, 1-Propinylcarbonylgruppe, 2-Propinylcarbonylgruppe, 1-Butinylcarbonylgruppe, 2-Butinylcarbonylgruppe, 3-Butinylcarbonylgruppe, 1-Methyl-2-propinylcarbonylgruppe, 1-Pentinylcarbonylgruppe, 2-Pentinylcarbonylgruppe, 3-Pentinylcarbonylgruppe, 4-Pentinylcarbonylgruppe, 1-Methyl-2-butinylcarbonylgruppe, 1-Methyl-3-butinylcarbonylgruppe, 2-Methyl-3-butinylcarbonylgruppe, 3-Methyl-1-Butinylcarbonylgruppe, 1,1-Dimethyl-2-propinylcarbonylgruppe, 2-Ethyl-2-propinylcarbonylgruppe, 1-Hexynilcarbonylgruppe, 2-Hexynilcarbonylgruppe, 3-Hexynilcarbonylgruppe, 4-Hexynilcarbonylgruppe, 5-Hexynilcarbonylgruppe, 1-Methyl-2-pentinylcarbonylgruppe, 1-Methyl-3-pentinylcarbonylgruppe, 1-Methyl-4-pentinylcarbonylgruppe, 2-Methyl-3-pentinylcarbonylgruppe, 2-Methyl-4-pentinylcarbonylgruppe, 3-Methyl-1-pentinylcarbonylgruppe, 3-Methyl-4-pentinylcarbonylgruppe, 4-Methyl-1-pentinylcarbonylgruppe, 4-Methyl-2-pentinylcarbonylgruppe, 1,1-Dimethyl-2-butinylcarbonylgruppe, 1,1-Dimethyl-3-butinylcarbonylgruppe, 1,2-Dimethyl-3-butinylcarbonylgruppe, 2,2-Dimethyl-3-butinylcarbonylgruppe, 3,3-Dimethyl-1-butinylcarbonylgruppe, 1-Ethyl-2-butinylcarbonylgruppe, 1-Ethyl-3-butinylcarbonylgruppe.

Eine Alkylcarbonsäureestergruppe besteht in dieser Spezifikation typischerweise aus einem Carbonsäureester, an dem eine Alkylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkylcarbonsäureestergruppe sind: Methylcarbonsäureestergruppe, Ethylcarbonsäureestergruppe, n-Propylcarbonsäureestergruppe, i-Propylcarbonsäureestergruppe, c-Propylcarbonsäureestergruppe, n-Butylcarbonsäureestergruppe, i-Butylcarbonsäureestergruppe, s-Butylcarbonsäureestergruppe, t-Butylcarbonsäureestergruppe, c-Butylcarbonsäureestergruppe, 1-Methyl-c-propylcarbonsäureestergruppe, 2-Methyl-c-propylcarbonsäureestergruppe, n-Pentylcarbonsäureestergruppe, 1-Methyl-n-butylcarbonsäureestergruppe, 2-Methyl-n-butylcarbonsäureestergruppe, 3 -Methyl-n-butylcarbonsäureestergruppe, 1,1-Dimethyl- n-propylcarbonsäureestergruppe, 1,2-Dimethyl-n-propylcarbonsäureestergruppe, 2,2-Dimethyl-n-propylcarbonsäureestergruppe, 1-Ethyl-n-propylcarbonsäureestergruppe, c-Pentylcarbonsäureestergruppe, 1-Methyl-c-butylcarbonsäureestergruppe, 2-Methyl-c-butylcarbonsäureestergruppe, 3-Methyl-c-butylcarbonsäureestergruppe, 1,2-Dimethyl-c-propylcarbonsäureestergruppe, 2,3-Dimethyl-c-propylcarbonsäureestergruppe, 1-Ethyl-c-propylcarbonsäureestergruppe, 2-Ethyl-c-propylcarbonsäureestergruppe, n-Hexylcarbonsäureestergruppe. 1-Methyl-n-pentylcarbonsäureestergruppe, 2-Methyl-n-pentylcarbonsäureestergruppe, 3-Methyl-n-pentylcarbonsäureestergruppe, 4-Methyl-n-pentylcarbonsäureestergruppe, 1,1-Dimethyl-n-butylcarbonsäureestergruppe, 1,2-Dimethyl-n-butylcarbonsäureestergruppe, 1,3-Dimethyl-n-butylcarbonsäureestergruppe, 2,2-Dimethyl-n-butylcarbonsäureestergruppe, 2,3-Dimethyl-n-butylcarbonsäureestergruppe, 3,3-Dimethyl- n-butylcarbonsäureestergruppe, 1-Ethyl-n-butylcarbonsäureestergruppe, 2-Ethyl-n-butylcarbonsäureestergruppe.

Eine Alkenylcarbonsäureestergruppe besteht in dieser Spezifikation typischerweise aus einem Carbonsäureester, an dem eine Alkenylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkenylcarbonsäureestergruppe sind: Ethenylcarbonsäureestergruppe, 1-Propenylcarbonsäureestergruppe, 2-Propenylcarbonsäureestergruppe, 1-Methyl-1-ethenylcarbonsäureestergruppe, 1-Butenylcarbonsäureestergruppe, 2-Butenylcarbonsäureestergruppe, 3-Butenylcarbonsäureestergruppe, 2-Methyl-1-propenylcarbonsäureestergruppe, 2-Methyl-2-propenylcarbonsäureestergruppe, 1-Ethylethenylcarbonsäureestergruppe, 1-Methyl-1-propenylcarbonsäureestergruppe, 1-Methyl-2-propenylcarbonsäureestergruppe, 1-Pentenylcarbonsäureestergruppe, 2-Pentenylcarbonsäureestergruppe, 3-Pentenylcarbonsäureestergruppe, 4-Pentenylcarbonsäureestergruppe, 1-n-Propylethenylcarbonsäureestergruppe, 1-Methyl-1-butenylcarbonsäureestergruppe, 1-Methyl-2-butenylcarbonsäureestergruppe, 1-Methyl-3 -butenylcarbonsäureestergruppe, 2-Ethyl-2-propenylcarbonsäureestergruppe, 2-Methyl-1-butenylcarbonsäureestergruppe, 2-Methyl-2-Butenylcarbonsäureestergruppe, 2-Methyl-3-butenylcarbonsäureestergruppe, 3-Methyl-1-butenylcarbonsäureestergruppe, 3-Methyl-2-butenylcarbonsäureestergruppe, 3 -Methyl-3 -butenylcarbonsäureestergruppe, 1,1-Dimethyl-2-propenylcarbonsäureestergruppe, 1-i-Propylethenylcarbonsäureestergruppe, 1,2-Dimethyl-1-propenylcarbonsäureestergruppe, 1,2-Dimethyl-2-propenylcarbonsäureestergruppe, 1-c-Pentenylcarbonsäureestergruppe, 2-c-Pentenylcarbonsäureestergruppe, 3-c-Pentenylcarbonsäureestergruppe, 1-Hexenylcarbonsäureestergruppe, 2-Hexenylcarbonsäureestergruppe, 3 -Hexenylcarbonsäureestergruppe, 4-Hexenylcarbonsäureestergruppe, 5-Hexenylcarbonsäureestergruppe, 1-Methyl-1 -pentenylcarbonsäureestergruppe, 1 -Methyl-2-Pentenylcarbonsäureestergruppe, 1 -Methyl-3 -pentenylcarbonsäureestergruppe, 1-Methyl-4-Pentenylcarbonsäureestergruppe, 1-n-Butylethenylcarbonsäureestergruppe, 2-Methyl-1-pentenylcarbonsäureestergruppe, 2-Methyl-2-pentenylcarbonsäureestergruppe, 2-Methyl-3 -pentenylcarbonsäureestergruppe, 2-Methyl-4-pentenylcarbonsäureestergruppe, 2-n-Propyl-2-propenylcarbonsäureestergruppe, 3-Methyl-1-pentenylcarbonsäureestergruppe, 3-Methyl-2-pentenylcarbonsäureestergruppe.

Eine Alkinylcarbonsäureestergruppe besteht in dieser Spezifikation typischerweise aus einem Carbonsäureester, an dem eine Alkinylgruppe, bestehend aus einem bis zu dreißig Kohlenstoffatomen, kovalent gebunden ist und die sowohl verzweigt, als auch unverzweigt sein kann. Bevorzugt besteht diese Kette aus einem bis zwanzig Kohlenstoffatomen. Beispiele für eine Alkinylcarbonsäureestergruppe sind: Ethinylcarbonsäureestergruppe, 1-Propinylcarbonsäureestergruppe, 2-Propinylcarbonsäureestergruppe, 1-Butinylcarbonsäureestergruppe, 2-Butinylcarbonsäureestergruppe, 3-Butinylcarbonsäureestergruppe, 1-Methyl-2-propinylcarbonsäureestergruppe, 1-Pentinylcarbonsäureestergruppe, 2-Pentinylcarbonsäureestergruppe, 3-Pentinylcarbonsäureestergruppe, 4-Pentinylcarbonsäureestergruppe, 1-Methyl-2-butinylcarbonsäureestergruppe, 1-Methyl-3-butinylcarbonsäureestergruppe, 2-Methyl-3 -butinylcarbonsäureestergruppe, 3-Methyl-1-Butinylcarbonsäureestergruppe, 1,1-Dimethyl- 2-propinylcarbonsäureestergruppe, 2-Ethyl-2-propinylcarbonsäureestergruppe, 1-Hexynilcarbonsäureestergruppe, 2-Hexynilcarbonsäureestergruppe, 3-Hexynilcarbonsäureestergruppe, 4-Hexynilcarbonsäureestergruppe, 5-Hexynilcarbonsäureestergruppe, 1-Methyl-2-pentinylcarbonsäureestergruppe, 1-Methyl-3-pentinylcarbonsäureestergruppe, 1-Methyl-4-pentinylcarbonsäureestergruppe, 2-Methyl-3-pentinylcarbonsäureestergruppe, 2-Methyl-4-pentinylcarbonsäureestergruppe, 3-Methyl-1-pentinylcarbonsäureestergruppe, 3-Methyl-4-pentinylcarbonsäureestergruppe, 4-Methyl-1-pentinylcarbonsäureestergruppe, 4-Methyl-2-pentinylcarbonsäureestergruppe, 1,1-Dimethyl-2-butinylcarbonsäureestergruppe, 1,1-Dimethyl-3-butinylcarbonsäureestergruppe, 1,2-Dimethyl-3-butinylcarbonsäureestergruppe, 2,2-Dimethyl-3-butinylcarbonsäureestergruppe, 3,3-Dimethyl-1-butinylcarbonsäureestergruppe, 1-Ethyl-2-butinylcarbonsäureestergruppe, 1-Ethyl-3-butinylcarbonsäureestergruppe.

In dieser Spezifikation kann eine Alkoxygruppe aus einer Alkyleinheit bestehen, die sowohl verzweigt als auch unverzweigt sein kann. Eine Alkoxygruppe besteht typischerweise aus einem bis zu dreißig Kohlenstoffatomen, bevorzugt aus einem bis zwanzig Kohlenstoffatomen. Beispiele für eine Alkoxygruppe sind: Methoxygruppe, Ethoxygruppe, Isopropoxygruppe, n-Butoxygruppe, sec.-Butoxygruppe, tert.-Butoxygruppe, Pentyloxygruppe, n-Hexyloxygruppe, n-Heptyloxygruppe, 2-Ethylhexyloxygruppe, n-Octyloxygruppe, n-Nonyloxygruppe, n-Decyloxygruppe, n-Tridecyloxygruppe, n-Tetradecyloxygruppe, n-Pentadecyloxygruppe, n-Hexadecyloxygruppe, n-Octadecyloxygruppe or Eicosyloxygruppe. Bevorzugt sind Alkoxygruppen mit einem bis zu sechs Kohlenstoffatomen in der Alkyleinheit.

Eine Cycloalkylgruppe, wie in dieser Spezifikation beschrieben, ist typischerweise eine cyclische Gruppe, bestehend aus fünf, sechs oder sieben Kohlenstoffatomen, die jeweils unabhängig voneinander substituiert sein können. Beispiele hierzu sind Alkylgruppen oder zwei Alkylgruppen, die zusammen mit den Ringkohlenstoffen, an denen sie gebunden sind, einen weiteren Ring bilden. Ein Beispiel für eine Cycloalkylgruppe ist eine Cyclohexylgruppe.

Eine Cycloalkoxygruppe, wie in dieser Spezifikation beschrieben, ist typischerweise eine cyclische Gruppe, bestehend aus fünf, sechs oder sieben Kohlenstoffatomen von denen minderstens eines kovalent an einem Sauerstoffatom gebunden ist. Diese Ringkohlenstoffatome können jeweils unabhängig voneinander substituiert sein, beispielsweise mit Alkylgruppen oder zwei Alkylgruppen, die zusammen mit den Ringkohlenstoffen an denen sie gebunden sind, einen weiteren Ring bilden. Ein Beispiel für eine Cycloalkoxygruppe ist eine Cyclohexyloxygruppe.

Eine Arylgruppe, wie in dieser Spezifikation beschrieben, ist typischerweise eine cyclische aromatische Gruppe, bestehend aus fünf bis zehn Kohlenstoffatomen, die jeweils unabhängig voneinander substituiert sein können. Beispiele hierzu sind Alkylgruppen oder zwei Alkylgruppen, die zusammen mit den Ringkohlenstoffen, an denen sie gebunden sind, einen weiteren Ring bilden. Beispiele für eine Arylgruppe sind Phenylgruppe, o-Biphenylylgruppe, m-Biphenylylgruppe, p-Biphenylylgruppe, 1-Anthrylgruppe, 2-Anthrylgruppe, 9-Anthrylgruppe, 1-Phenantolylgruppe, 2-Phenantolylgruppe, 3-Phenantolylgruppe, 4-Phenantolylgruppe, 9-Phenantolylgruppe.

Eine Heteroarylgruppe, wie in dieser Spezifikation beschrieben, ist typischerweise eine cyclische aromatische Gruppe bestehend aus vier bis zehn Kohlenstoffatomen und mindestens einem Heteroatom, die jeweils unabhängig voneinander substituiert sein können. Beispiele hierzu sind Alkylgruppen, oder zwei Alkylgruppen die zusammen mit den Ringkohlenstoffen an denen sie gebunden sind einen weiteren Ring bilden. Beispiele für Heteroatome sind hierbei ein Sauerstoffatom, Stickstoffatom, Phosphoratom, Boratom, Selenatom oder ein Schwefelatom. Beispiele für eine Heteroarylgruppe sind Furylgruppe, Thienylgruppe, Pyrrolylgruppe oder Imidazolylgruppe.

Eine Aryloxylgruppe, wie in dieser Spezifikation beschrieben, ist typischerweise eine Arylgruppe, wobei Aryl zuvor bereits definiert wurde, die kovalent an ein Sauerstoffatom gebunden ist. Beispiele für eine Aryloxylgruppe sind Phenyloxyl oder Naphthyloxyl.

Eine Aralkylgruppe, wie in dieser Spezifikation beschrieben, ist typischerweise eine Arylgruppe, wobei Aryl zuvor bereits definiert wurde, die kovalent an eine Alkylgruppe gebunden ist. Diese Gruppe kann beispielsweise mit Alkylgruppen oder Halogenatomen substituiert sein. Ein Beispiel für eine Aralylgruppe ist Benzyl.

Die durchschnittliche Molmasse (Mₙ) der oligomeren oder polymeren Verbindung dieser Erfindung liegt im Bereich 800 bis 4000000 g/mol, bevorzugt im Bereich von 2000 bis 2000000 g/mol, besonderes bevorzugt im Bereich von 4000 bis 400000 g/mol. Die durchschnittliche Molmasse wird mittels Größenausschlusschromatogaphie (Polystyrolstandard) bestimmt.

Im Allgemeinen besteht die oligomere oder polymere Verbindung der erfindungsgemäßen allgemeinen Formel I dieser Erfindung aus 2 und 5000 Wiederholeinheiten, bevorzugt aus 10 bis 1000 Wiederholeinheiten.

Die oligomeren oder polymeren Verbindungen der erfindungsgemäßen allgemeinen Formel I dieser Erfindung können sowohl Homopolymere als auch Copolymere sein. Homopolymere sind Polymere, die nur aus einem Monomer synthetisiert wurden. Copolymere sind Polymere, die aus zwei oder mehreren Polymeren synthetisiert wurden. Werden zwei oder mehrere Monomere bei der Synthese verwendet, so können die Monomere der Wiederholeinheiten der oligomeren oder polymeren Verbindung dieser Erfindung sowohl in statistischer Verteilung, als Blöcke oder alternierend in der oligomeren oder polymeren Verbindung vorliegen. Die oligomeren oder polymeren Verbindungen dieser Erfindung können sowohl in linearer Form als auch als quervernetzt sein. Eine Quervernetzung kann beispielweise durch die Copolymerisation mit einem geringen Anteils eines organischen Molekül mit zwei polymerisierbaren Gruppen, bevorzugt einem höher funktionalisierten Monomer, erfolgen.

Die oligomeren oder polymeren Verbindungen der erfindungsgemäßen allgemeinen Formel I werden durch die Polymerisation einer 9,10-Bis(1,3-dithiol-2-yliden)-9,10-dihydroanthracenverbindung der allgemeinen Formel I' synthetisiert.

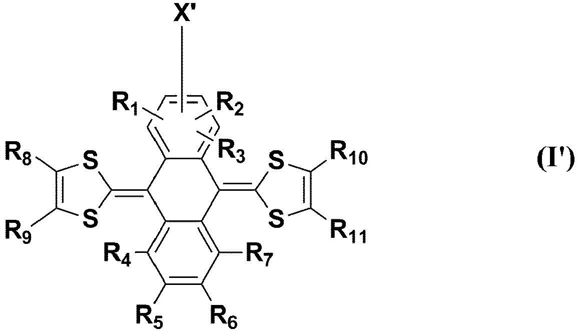

X' ist dabei eine organische polymerisierbare Gruppe, die typischerweise aus einer organischen Doppelbindung, oder einer organischen Dreifachbindung, oder einem Oxiran oder einem Aziridin besteht. Besonders bevorzugt sind dabei organische polymerisierbare Gruppen, die in den Formeln II' bis VIII' dargestellt sind.

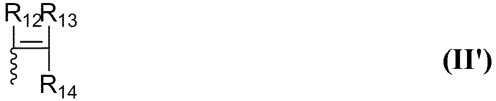

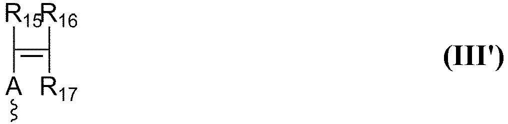

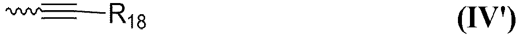

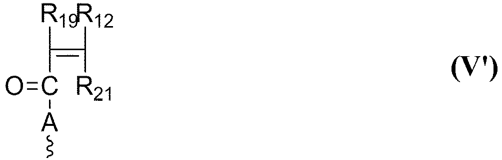

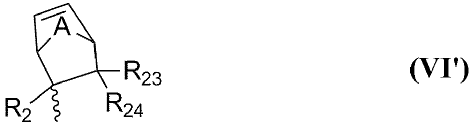

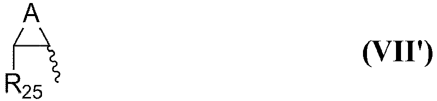

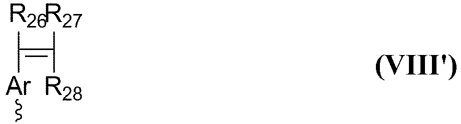

Mit wiederum den bereits vorbeschriebenen Definitionen für R₁₂ bis R₂₈, A und Ar.

Eine 9,10-Bis(1,3-dithiol-2-yliden)-9,10-dihydroanthracenverbindung der allgemeinen Formel I' kann durch die Kombination bekannter Reaktionen hergestellt werden.

Die Herstellung der Verbindung der allgemeinen Formel I' ist in nachfolgenden Schemata 1-7 dargestellt, jedoch nicht auf diese beschränkt.

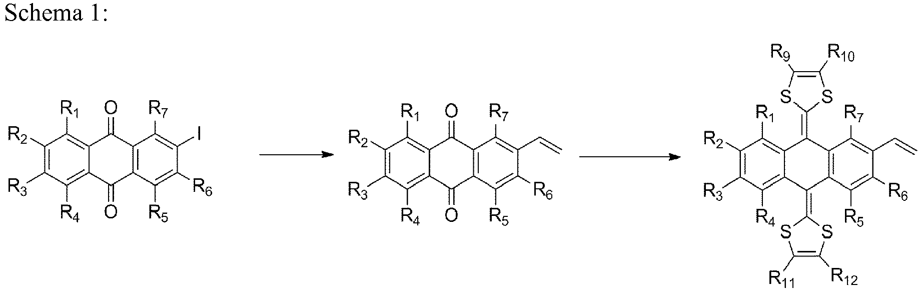

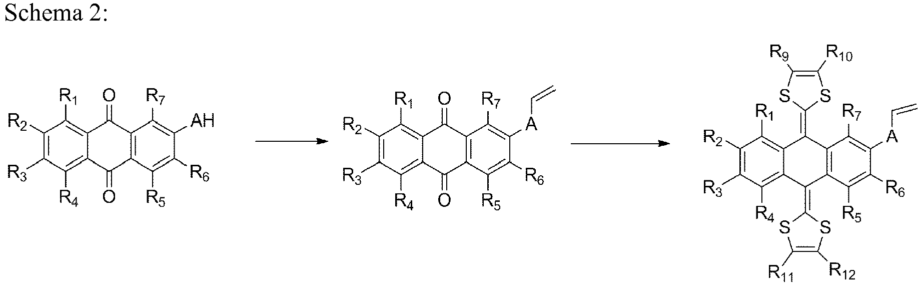

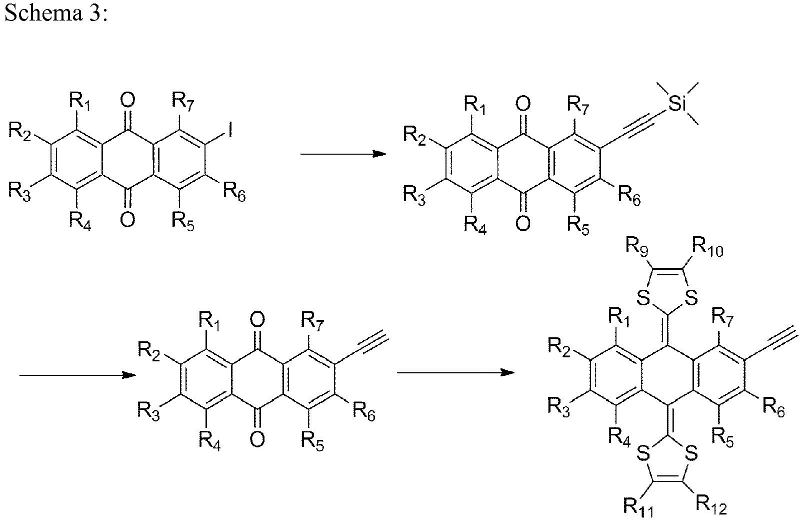

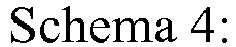

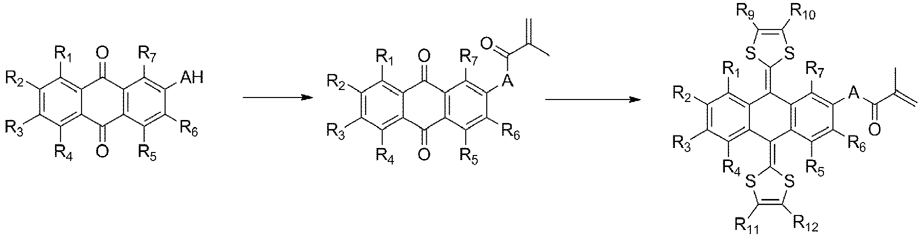

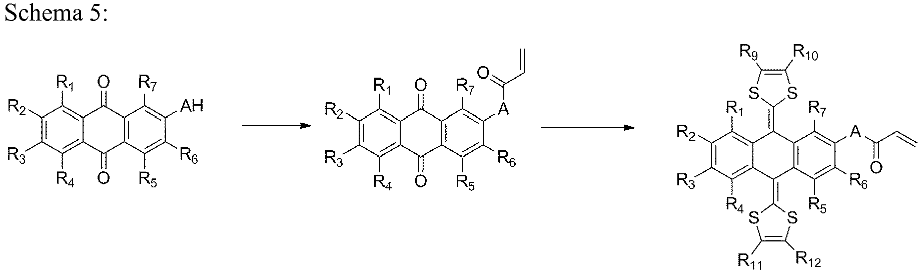

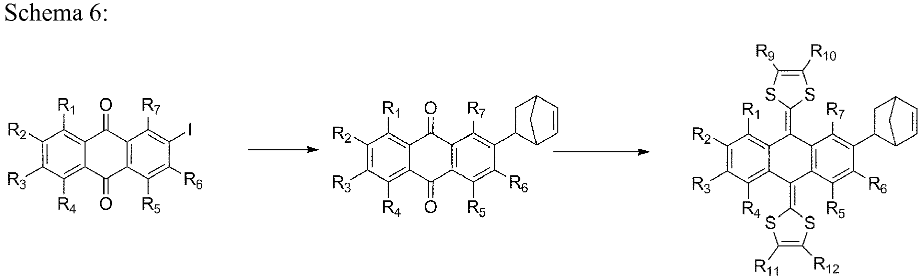

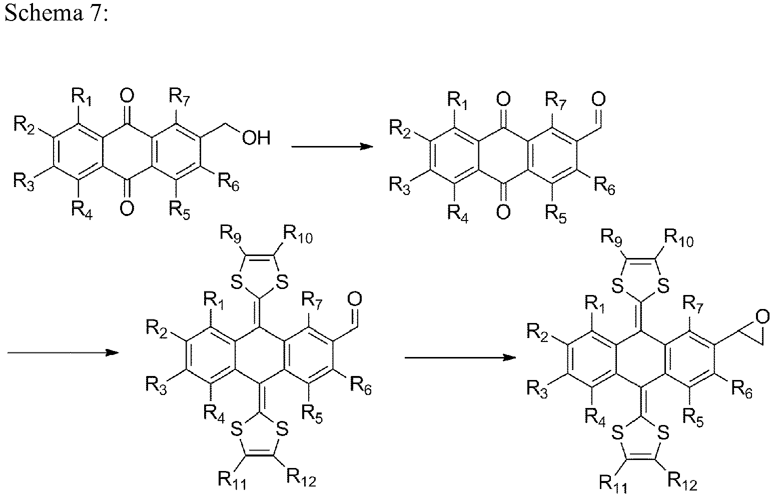

An sich bekannte Methoden sind ausreichend, um die Verbindung der oben genannten allgemeinen Formel I' gemäß den vorstehenden Schemata 1-7 zu synthetisieren.

Für den Fall, dass X' in der allgemeinen Formel I' der oben genannten Formel II' entspricht, kann die Verbindung der Formel I' nach einer bekannten Methode der Synthese von Polystyrol und deren Abwandlungen synthetisiert werden. Bevorzugt wird die oben genannte Verbindung I' durch radikalische Polymerisation, wie beispielsweise freie radikalische Polymerisation, aber auch eine kontrollierte radikalische Polymerisationsmethode, wie beispielsweise, Reversible Addition-Fragmentation Chain Transfer Polymerisation (RAFT), Atom-Transfer Radical Polymerisation (ATRP) oder Nitroxide-Mediated Polymerisation (NMP), in einem Temperaturbereich von -30 bis 150 °C, vorteilhaft in einem Temperaturbereich von 40 bis 120 °C, in einem Lösungsmittel und in einer Reaktionszeit von 0,1 bis 100 Stunden unter der Verwendung eines Initiators, wie beispielsweise Azoverbindungen oder Peroxide, bevorzugt Benzoylperoxid oder 2,2'-Azobisisobutyrolnitril, synthetisiert. Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Ebenfalls bevorzugt wird die oben genannte Verbindung I' durch kationische Polymerisation in einem Temperaturbereich von -30 bis 150 °C, vorteilhaft in einem Temperaturbereich von - 20 bis 50 °C, in einem Lösungsmittel und einer Reaktionszeit von 0,1 bis 100 Stunden unter der Verwendung eines Katalysators, wie beispielsweise Lewissäuren oder Protonensäuren, bevorzugt Schwefelsäure, Salpetersäure, Perchlorsäure, Bortrifluoretherat Komplex, Aluminiumtrichlorid, Zinntetrachlorid oder Titantetrachlorid, synthetisiert. Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, Tetrahydrofuran, 1,4-Dioxolan, 1,2-Dichlorethan, Toluol, Xylol, Chlorbenzol oder o-Dichlorbenzol.

Ebenfalls bevorzugt wird die oben genannte Verbindung I' durch anionische Polymerisation in einem Temperaturbereich von -78 bis 150 °C, vorteilhaft in einem Temperaturbereich von - 50 bis 50 °C, in einem Lösungsmittel und einer Reaktionszeit von 0,1 bis 100 Stunden unter der Verwendung eines Katalysators, wie beispielsweise Lewisbasen oder Basen, bevorzugt Metallamide, wie Natriumamid und LiC₂H₅, Alkoxiden wie Methanolat oder Ethanolat, Hydroxide wie Natriumhydroxyd oder Kaliumhydroxyd, Cyanide, Phosphine, Amine oder metallorganische Verbindungen wie beispielsweise n-Buthyllithium oder Vinylmagnesiumbromid synthetisiert. Für verwendete Lösungsmittel bestehen kaum Einschränkungen. Bevorzugt sind organische Lösungsmittel, wie beispielsweise, Tetrahydrofuran, 1,4-Dioxolan, Diethylether, tert-Butylmethylether, Toluol, Xylol, Chlorbenzol oder o-Dichlorbenzol.

Ebenfalls bevorzugt wird die oben genannte Verbindung I' durch anionische Gruppentransfer Polymerisation in einem Temperaturbereich von -30 bis 150 °C, vorteilhaft in einem Temperaturbereich von -20 bis 50 °C, in einem Lösungsmittel und einer Reaktionszeit von 0,1 bis 100 Stunden unter der Verwendung eines Initiator wie beispielsweise eines Silylketenacetal und unter Verwendung eines Katalysators, wie beispielsweise anorganische Salze, bevorzugt Fluoride, Azide oder Cyanide oder Lewissäuren, bevorzugt Zinkchlorid oder Aluminiumdialkylchlorid, synthetisiert. Für verwendete Lösungsmittel bestehen kaum Einschränkungen. Bevorzugt sind organische Lösungsmittel, wie beispielsweise, N,N'-Dimethylformamid, N,N'-Dimethylacetamid, Dimethylsulfoxid, N-Methylpyrrolidin, Dichlormethan, 1,2-Dichlorethan, Tetrahydrofuran, 1,4-Dioxolan, Diethylether, tert-Butylmethylether, Toluol, Xylol, Chlorbenzol oder o-Dichlorbenzol.

Für den Fall, dass X' in der allgemeinen Formel I' der oben genannten Formel III' entspricht, kann die Verbindung der Formel I' nach einer bekannten Methode der Synthese von Polyvinylether und deren Abwandlungen synthetisiert werden. Bevorzugt wird die oben genannte Verbindung I' durch kationische Polymerisation in einem Temperaturbereich von - 30 bis 150 °C, vorteilhaft in einem Temperaturbereich von -20 bis 50 °C, in einem Lösungsmittel und einer Reaktionszeit von 0,1 bis 100 Stunden unter der Verwendung eines Katalysators, wie beispielsweise Lewissäuren oder Protonensäuren, bevorzugt Schwefelsäure, Salpetersäure, Perchlorsäure, Bortrifluoretherat Komplex, Aluminiumtrichlorid, Zinntetrachlorid oder Titantetrachlorid, synthetisiert.
Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, Tetrahydrofuran, 1,4-Dioxolan, 1,2-Dichlorethan, Toluol, Xylol, Chlorbenzol oder o-Dichlorbenzol.

Für den Fall, dass X' in der allgemeinen Formel I' der oben genannten Formel IV' entspricht, kann die Verbindung der Formel I' nach einer bekannten Methode der Synthese von Polyacetylenen und deren Abwandlungen synthetisiert werden. Bevorzugt wird die oben genannte Verbindung I' durch metallkatalysierte Polymerisation in einem Temperaturbereich von -30 bis 150 °C, vorteilhaft in einem Temperaturbereich von 0 bis 100 °C, in einem Lösungsmittel und in einer Reaktionszeit von 0,1 bis 100 Stunden unter der Verwendung eines Katalysators, wie beispielsweise einem Wilkinson Katalysator, einem Ziegler-Natta Katalysator, einem Luttinger Katalysator, einem Molybdän Komplex, einem Wolframkomplex, einem Rhodiumkomplex oder einer elektrochemischen Polymerisationsmethode unter Verwendung von Nickelbromid, synthetisiert.
Für verwendete Lösungsmittel bestehen kaum Einschränkungen. Bevorzugt sind organische Lösungsmittel wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X' in der allgemeinen Formel I' der oben genannten Formel V' entspricht, kann die Verbindung der Formel I' nach einer bekannten Methode der Synthese von Polyacrylaten und deren Abwandlungen synthetisiert werden. Bevorzugt wird die oben genannte Verbindung I' durch freie radikalische Polymerisation, aber auch kontrollierte radikalische Polymerisationsmethoden, wie beispielsweise, Reversible Addition-Fragmentation Chain Transfer Polymerisation (RAFT), Atom-Transfer Radical Polymerisation (ATRP), Cobalt vermittelte radikalische Polymerisation (CMRP) oder Nitroxide-Mediated Polymerisation (NMP), in einem Temperaturbereich von -30 bis 150 °C, vorteilhaft in einem Temperaturbereich von 40 bis 120 °C, in einem Lösungsmittel und in einer Reaktionszeit von 0,1 bis 100 Stunden unter der Verwendung eines Initiators, wie beispielsweise Azoverbindungen oder Peroxiden, bevorzugt Benzoylperoxid oder 2,2'-Azobisisobutyrolnitril, synthetisiert.
Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Ebenfalls bevorzugt wird die oben genannte Verbindung I' durch anionische Polymerisation in einem Temperaturbereich von -78 bis 150 °C, vorteilhaft in einem Temperaturbereich von - 50 bis 50 °C, in einem Lösungsmittel und einer Reaktionszeit von 0,1 bis 100 Stunden unter der Verwendung eines Katalysators, wie beispielsweise Lewisbasen oder Basen, bevorzugt Metallamide, wie Natriumamid und LiC₂H₅, Alkoxiden wie Methanolat oder Ethanolat, Hydroxide wie Natriumhydroxyd oder Kaliumhydroxyd, Cyanide, Phosphine, Amine oder metallorganische Verbindungen wie beispielsweise n-Buthyllithium oder Vinylmagnesiumbromid synthetisiert. Für verwendete Lösungsmittel bestehen kaum Einschränkungen. Bevorzugt sind organische Lösungsmittel, wie beispielsweise, Tetrahydrofuran, 1,4-Dioxolan, Diethylether, tert-Butylmethylether, Toluol, Xylol, Chlorbenzol oder o-Dichlorbenzol.

Ebenfalls bevorzugt wird die oben genannte Verbindung I' durch anionische Gruppentransfer Polymerisation in einem Temperaturbereich von -78 bis 150 °C, vorteilhaft in einem Temperaturbereich von -20 bis 50 °C, in einem Lösungsmittel und einer Reaktionszeit von 0,1 bis 100 Stunden unter der Verwendung eines Initiator wie beispielsweise eines Silylketenacetal und unter Verwendung eines Katalysators, wie beispielsweise anorganische Salze, bevorzugt Fluoride, Azide oder Cyanide oder Lewissäuren, bevorzugt Zinkchlorid oder Aluminiumdialkylchlorid, synthetisiert. Für verwendete Lösungsmittel bestehen kaum Einschränkungen. Bevorzugt sind organische Lösungsmittel, wie beispielsweise, N,N'-Dimethylformamid, N,N'-Dimethylacetamid, Dimethylsulfoxid, N-Methylpyrrolidin, Dichlormethan, 1,2-Dichlorethan, Tetrahydrofuran, 1,4-Dioxolan, Diethylether, tert-Butylmethylether, Toluol, Xylol, Chlorbenzol oder o-Dichlorbenzol.

Für den Fall, dass X' in der allgemeinen Formel I' der oben genannten Formel VI' entspricht, kann die Verbindung der Formel I' nach einer bekannten Methode der Synthese von Polynorbornenen und deren Abwandlungen synthetisiert werden. Bevorzugt wird die oben genannte Verbindung I' durch metalkatalysierte Polymerisation in einem Temperaturbereich von -30 bis 150 °C, vorteilhaft in einem Temperaturbereich von 0 bis 100 °C, in einem Lösungsmittel und in einer Reaktionszeit von 0,1 bis 100 Stunden unter der Verwendung eines Katalysators, wie beispielsweise einem Grubbs Katalysator, einem Molybdänkomplex, einem Wolframkomplex oder einem Rutheniumkomplex, synthetisiert.
Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Tetrahydrofuran, Toluol, Xylol, Chlorbenzol oder o-Dichlorbenzol.

Für den Fall, dass X' in der allgemeinen Formel I' der oben genannten Formel VII' entspricht, kann die Verbindung der Formel I' nach einer bekannten Methode der Synthese von Polyethylengylkol und deren Abwandlungen synthetisiert werden.
Bevorzugt wird die oben genannte Verbindung I' durch kationische Polymerisation in einem Temperaturbereich von -30 bis 150 °C, vorteilhaft in einem Temperaturbereich von 40 bis 120 °C, in einem Lösungsmittel und einer Reaktionszeit von 0,1 bis 100 Stunden unter der Verwendung eines Katalysators, wie beispielsweise Lewissäuren oder Protonensäuren, bevorzugt Schwefelsäure, Salpetersäure, Perchlorsäure, Bortrifluoretheratkomplex, Aluminiumtrichlorid, Zinntetrachlorid, Diethylzink/Wasser oder Titantetrachlorid, synthetisiert.
Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, Tetrahydrofuran, 1,4-Dioxolan, 1,2-Dichlorethan, Toluol, Xylol, Chlorbenzol oder o-Dichlorbenzol.

Ebenfalls bevorzugt wird die oben genannte Verbindung I' durch anionische Polymerisation in einem Temperaturbereich von -78 bis 150 °C, vorteilhaft in einem Temperaturbereich von - 50 bis 50 °C, in einem Lösungsmittel und einer Reaktionszeit von 0,1 bis 100 Stunden unter der Verwendung eines Katalysators, wie beispielsweise Lewisbasen oder Basen, bevorzugt Metallamide, wie Natriumamid und LiC₂H₅, Alkoxiden wie Methanolat oder Ethanolat, Hydroxide wie Natriumhydroxyd oder Kaliumhydroxyd, Cyanide, Phosphine, Amine oder metallorganische Verbindungen wie beispielsweise n-Buthyllithium oder Vinylmagnesiumbromid, synthetisiert. Für verwendete Lösungsmittel bestehen kaum Einschränkungen. Bevorzugt sind organische Lösungsmittel, wie beispielsweise, Tetrahydrofuran, 1,4-Dioxolan, Diethylether, tert-Butylmethylether, Toluol, Xylol, Chlorbenzol oder o-Dichlorbenzol.

Für den Fall, dass X' in der allgemeinen Formel I' der oben genannten Formel VIII' entspricht, kann die Verbindung der Formel I' nach einer bekannten Methode der Synthese von Polystyrol und deren Abwandlungen synthetisiert werden. Bevorzugt wird die oben genannte Verbindung I' durch radikalische Polymerisation, wie beispielsweise freie radikalische Polymerisation, aber auch kontrollierte radikalische Polymerisationsmethode, wie beispielsweise, Reversible Addition-Fragmentation Chain Transfer Polymerisation (RAFT), Atom-Transfer Radical Polymerisation (ATRP), oder Nitroxide-Mediated Polymerisation (NMP), in einem Temperaturbereich von -30 bis 150 °C, vorteilhaft in einem Temperaturbereich von 40 bis 120 °C, in einem Lösungsmittel und in einer Reaktionszeit von 0,1 bis 100 Stunden unter der Verwendung eines Initiators, wie beispielsweise Azoverbindungen oder Peroxide, bevorzugt Benzoylperoxid oder 2,2'-Azobisisobutyrolnitril, synthetisiert. Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Ebenfalls bevorzugt wird die oben genannte Verbindung I' durch kationische Polymerisation in einem Temperaturbereich von -30 bis 150 °C, vorteilhaft in einem Temperaturbereich von - 20 bis 50 °C, in einem Lösungsmittel und einer Reaktionszeit von 0,1 bis 100 Stunden unter der Verwendung eines Katalysators, wie beispielsweise Lewissäuren oder Protonensäuren, bevorzugt Schwefelsäure, Salpetersäure, Perchlorsäure, Bortrifluoretherat Komplex, Aluminiumtrichlorid, Zinntetrachlorid oder Titantetrachlorid, synthetisiert. Für verwendete Lösungsmittel bestehen kaum Einschränkungen. Bevorzugt sind organische Lösungsmittel, wie beispielsweise N,N'-Dimethylformamid, N,N'-Dimethylacetamid, Dimethylsulfoxid, N-Methylpyrrolidin, Dichlormethan, Tetrahydrofuran, 1,4-Dioxolan, 1,2-Dichlorethan, Toluol, Xylol, Chlorbenzol oder o-Dichlorbenzol.

Ebenfalls bevorzugt wird die oben genannte Verbindung I' durch anionische Polymerisation in einem Temperaturbereich von -78 bis 150 °C, vorteilhaft in einem Temperaturbereich von - 50 bis 50 °C, in einem Lösungsmittel und einer Reaktionszeit von 0,1 bis 100 Stunden unter der Verwendung eines Katalysators, wie beispielsweise Lewisbasen oder Basen, bevorzugt Metallamide, wie Natriumamid und LiC₂H₅, Alkoxiden wie Methanolat oder Ethanolat, Hydroxide wie Natriumhydroxyd oder Kaliumhydroxyd, Cyanide, Phosphine, Amine oder metallorganische Verbindungen wie beispielsweise n-Buthyllithium oder Vinylmagnesiumbromid, synthetisiert. Für verwendete Lösungsmittel bestehen kaum Einschränkungen. Bevorzugt sind organische Lösungsmittel, wie beispielsweise, Tetrahydrofuran, 1,4-Dioxolan, Diethylether, tert-Butylmethylether, Toluol, Xylol, Chlorbenzol oder o-Dichlorbenzol.

Ebenfalls bevorzugt wird die oben genannte Verbindung I' durch anionische Gruppentransfer Polymerisation in einem Temperaturbereich von -78 bis 150 °C, vorteilhaft in einem Temperaturbereich von -20 bis 50 °C, in einem Lösungsmittel und einer Reaktionszeit von 0,1 bis 100 Stunden unter der Verwendung eines Initiators wie beispielsweise eines Silylketenacetal sowie unter Verwendung eines Katalysators, wie beispielsweise anorganische Salze, bevorzugt Fluoride, Azide oder Cyanide oder Lewissäuren, bevorzugt Zinkchlorid oder Aluminiumdialkylchlorid, synthetisiert. Für verwendete Lösungsmittel bestehen kaum Einschränkungen. Bevorzugt sind organische Lösungsmittel, wie beispielsweise, N,N'-Dimethylformamid, N,N'-Dimethylacetamid, Dimethylsulfoxid, N-Methylpyrrolidin, Dichlormethan, 1,2-Dichlorethan, Tetrahydrofuran, 1,4-Dioxolan, Diethylether, tert-Butylmethylether, Toluol, Xylol, Chlorbenzol oder o-Dichlorbenzol.

Außerdem werden die oligomeren oder polymeren Verbindungen der erfindungsgemäßen allgemeinen Formell durch polymeranaloge Umsetzung einer ,10-Bis(1,3-dithiol-2-yliden)-9,10-dihydroanthracenverbindung der allgemeinen Formel I" mit einer oligomeren oder polymeren Verbindung der allgemeinen Formel P' synthetisiert. 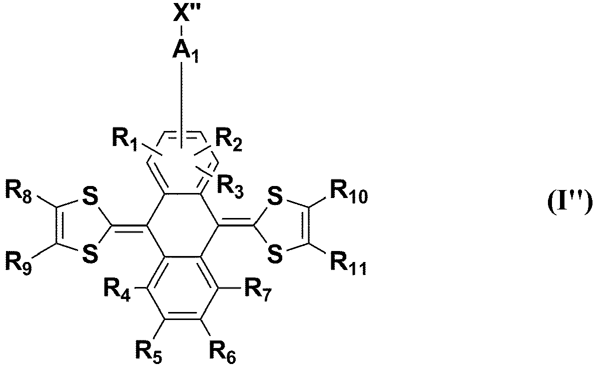 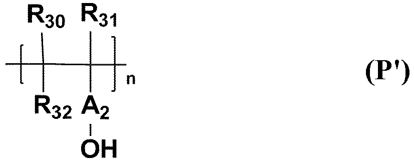 wobei
- R₁ bis R₇ und R₃₀ bis R₃₂:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens fünf der Substituenten R₁ bis R₇, und Nicht-Wasserstoffatome, vorzugsweise Halogenatome, Alkylgruppen, Alkoxygruppen, Cyanogruppen und/oder Nitrogruppen als null bis zwei der Substituenten R₁ bis R₇ sind, und/oder Wasserstoffatome als mindestens zwei der Substituenten R₃₀ bis R₃₂ und Nicht-Wasserstoffatome, vorzugsweise Halogenatome, Alkylgruppen, Alkoxygruppen, Cyanogruppen und/oder Nitrogruppen, als null bis einem der Substituenten R₃₀ bis R₃₂ besonders bevorzugt sind,
- R₈ bis R₁₁:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei die Substituenten R₈ und R₉ bzw. die Substituenten R₁₀ und R₁₁ einen weiteren Ring bilden können, der aus fünf bis sieben Atomen besteht (der Ring kann aromatisch, heteroaromatisch, oder nicht aromatisch sein; ist der Ring nicht aromatisch, so kann er aus verschiedenen Gruppen, wie beispielsweise Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminoruppen, Monoalkylaminogruppen, Dialkylaminogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, bestehen; besonders bevorzugt entsprechen sich R₈ bis R₁₁ und sind Alkylgruppen, wie typischerweise Methylgruppen oder Ethylgruppen, Alkylthiogruppen, wie Methylthiogruppen, oder Ethylthiogruppen und Thiolgruppen),
- X":: eine elektrophile organische Gruppe ist, welche nukleophil von der Hydoxygruppe der Verbindung P' angegriffen wird und somit eine kovalente Bindung zwischen der Verbindung I" und P' bildet (bevorzugt ist X" eine Isocyanatgruppe, eine Carbonsäurehalogenidgruppe, wobei das Halogen vorzugsweise Chlor, Brom oder Iod ist, eine Carbonsäuregruppe, ein Halogenatom, wobei das Halogen bevorzugt Chlor, Brom oder Iod ist, oder eine Carbonylgruppe, eine Anhydridgruppe),
- A₁ und A₂:: eine kovalente Bindung, eine Alkylgruppe, eine Alkenylgruppe, eine Alkinylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe, wobei eine kovalente Bindung oder eine Alkylgruppe als A₁ und A₂ besonders vorteilhaft ist,
- n:: eine Ganzzahl größer gleich 2 ist.

Die Herstellung der Verbindung der erfindungsgemäßen allgemeinen Formel I durch polymeranaloge Reaktion aus den oben genannten Verbindungen I" und P' und deren Herstellung ist in nachfolgenden Schemata 8-13 dargestellt, jedoch nicht auf diese beschränkt. 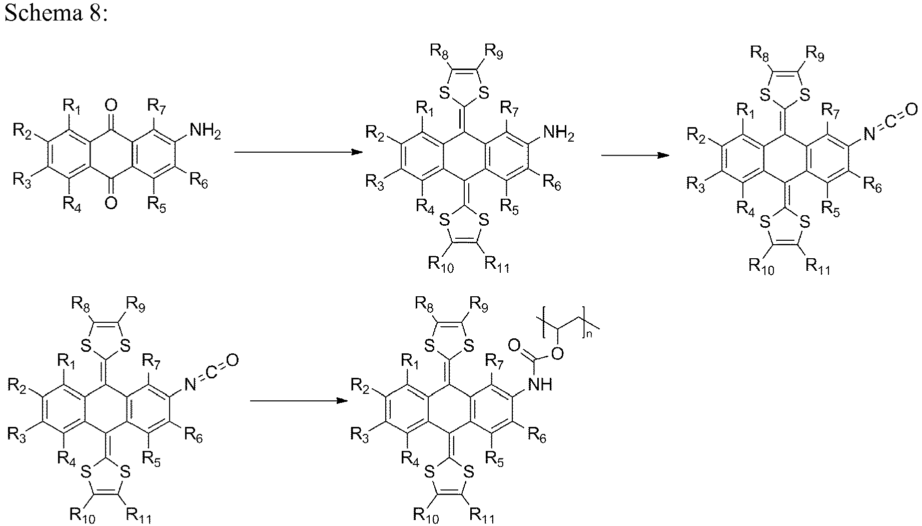 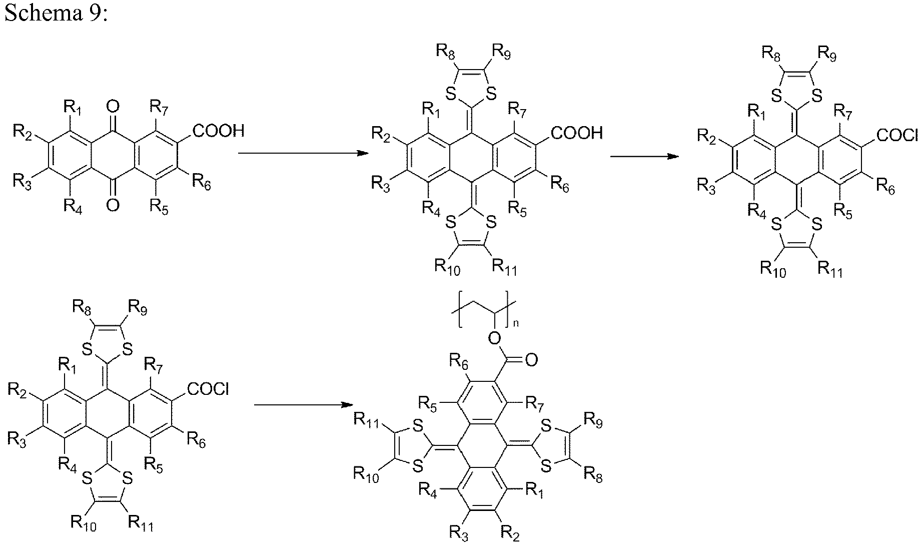 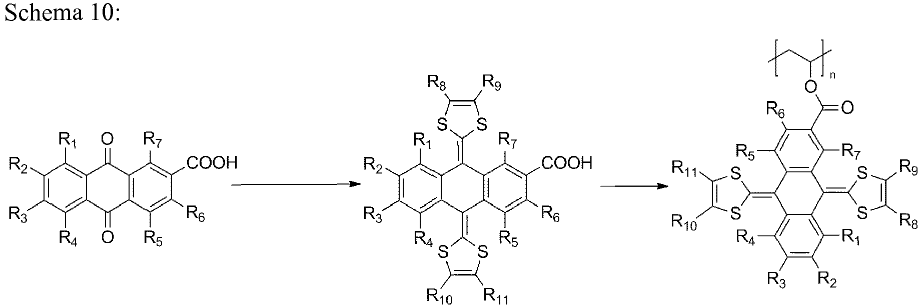 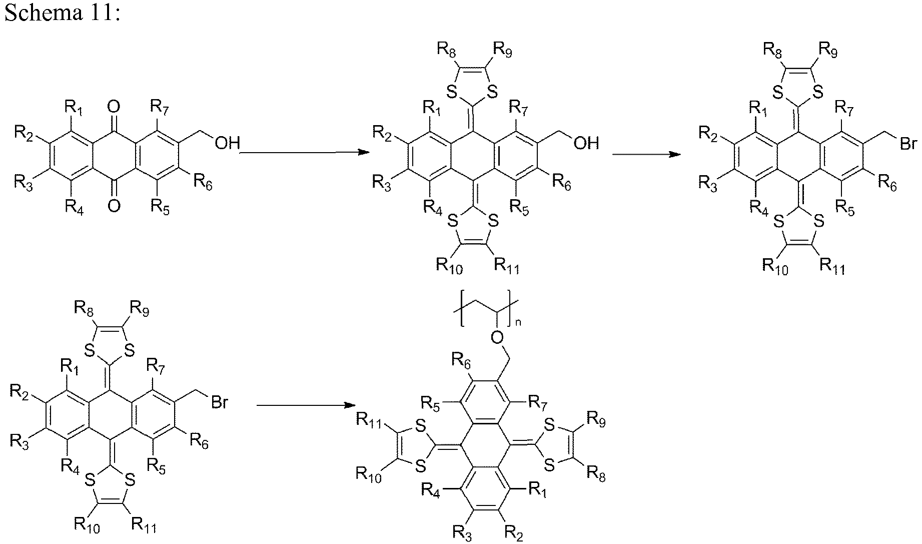 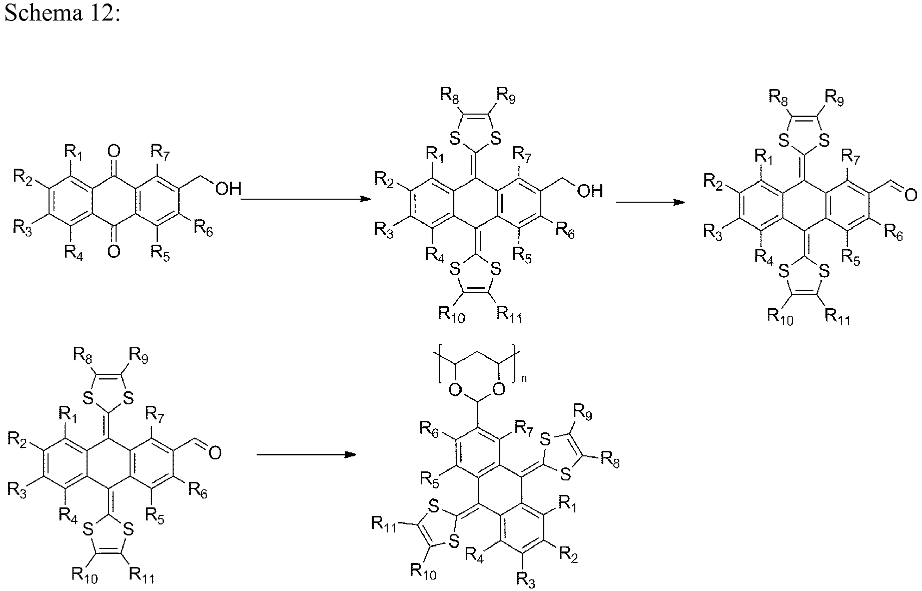 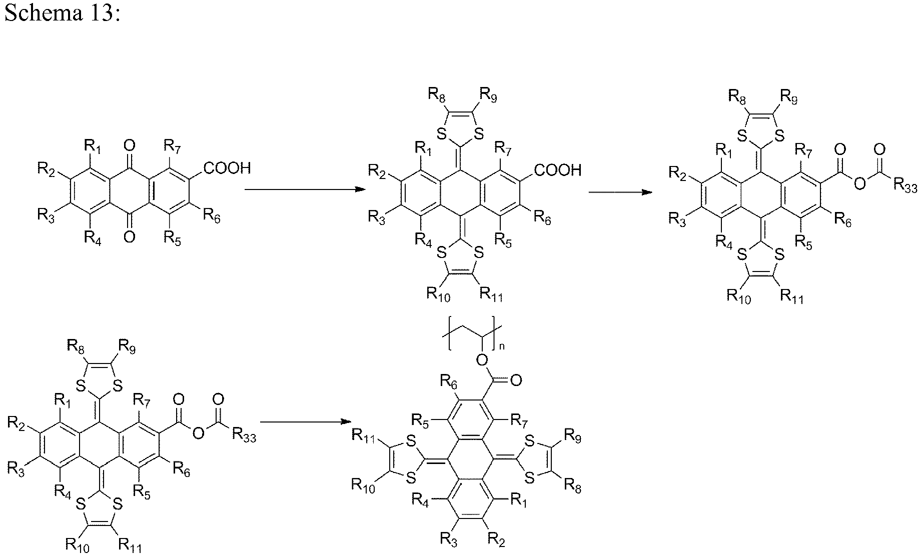 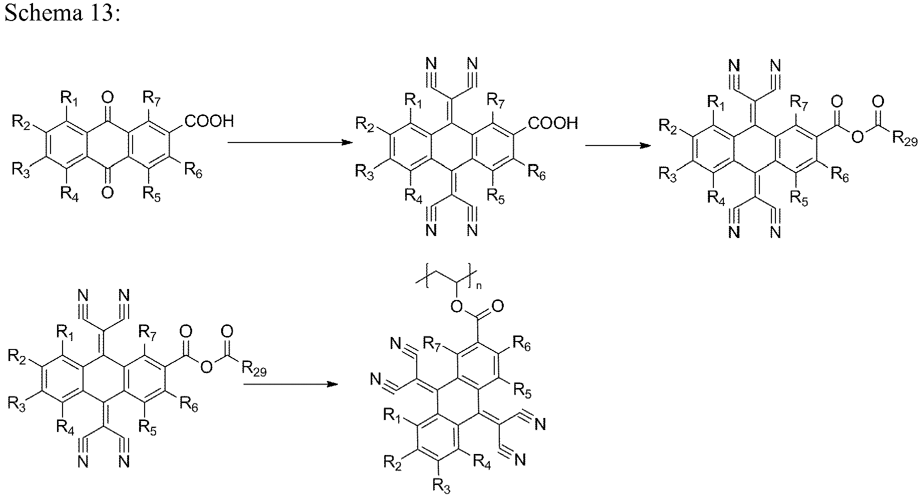 R₃₃ ist bevorzugt ein Wasserstoffatome, eine Alkylgruppe, eine Alkenylgruppe, eine Alkinylgruppe, eine Alkoxygruppe, eine Alkylthiogruppe, eine Cycloalkylgruppe, eine Cycloalkoxygruppe, eine Arylgruppe, eine Heteroarylgruppe, eine Aryloxygruppe, eine Aralkylgruppe. Besonders bevorzugt ist R₃₃ eine Alkylgruppe.

An sich bekannte Methoden sind ausreichend, um die Verbindung der oben genannten erfindungsgemäßen allgemeinen Formel I gemäß nach den Schemata 8-13 zu synthetisieren.

Für den Fall, dass X" in der allgemeinen Formel I" einer Isocyanatgruppe entspricht, kann die Verbindung der Formel I durch Reaktion der Verbindung I" mit der Verbindung P' nach einer bekannten Methode der Synthese von Urethanen und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X" in der allgemeinen Formel I" einer Carbonsäurehalogenidgruppe entspricht, kann die Verbindung der Formel I durch Reaktion der Verbindung I" mit der Verbindung P' nach einer bekannten Methode der Synthese von Carbonsäureester und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden unter der Verwendung eines Katalysators, wie beispielsweise eines Pyridinderivates, wie typischerweise 4-(Dimethylamino)pyridine, oder eines Carbodiimidderivates wie typischerweise N,N'-Dicyclohexylcarbodiimide synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X" in der allgemeinen Formel I" einer Carbonsäuregruppe entspricht, kann die Verbindung der Formel I durch Reaktion der Verbindung I" mit der Verbindung P' nach einer bekannten Methode der Synthese von Carbonsäureester und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden unter der Verwendung eines Katalysators, wie beispielsweise eines Pyridinderivates, wie typischerweise 4-(Dimethylamino)pyridine, oder eines Carbodiimidderivates, wie typischerweise N,N'-Dicyclohexylcarbodiimide, synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X" in der allgemeinen Formel I" einem Halogenatom entspricht, kann die Verbindung der Formel I durch Reaktion der Verbindung I" mit der Verbindung P' nach einer bekannten Methode der Synthese von Ethern und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden unter der Verwendung eines Katalysators, wie beispielsweise Base wie Natriumhydrid, Natriumhydroxid, Kalium tert-Butylat, DBU, oder DBN synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X" in der allgemeinen Formel I" einer Carbonylgruppe entspricht, kann die Verbindung der Formel I durch Reaktion der Verbindung I" mit der Verbindung P' nach einer bekannten Methode der Synthese von Acetalen und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden unter der Verwendung eines Katalysators, wie beispielsweise Protonensäure wie p-Toluolsulfonsäure, Salzsäure, Schwefelsäure, oder Trifluoressigsäure synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Für den Fall, dass X" in der allgemeinen Formel I" einer Anhydridgruppe entspricht, kann die Verbindung der Formel I durch Reaktion der Verbindung I" mit der Verbindung P' nach einer bekannten Methode der Synthese von Carbonsäureester und deren Abwandlungen synthetisiert werden. Bevorzugt wird die oben genannte Verbindung I in einem Temperaturbereich von -78 bis 150 °C, vorteilhaft in einem Temperaturbereich von -40 bis 120 °C, in einem Lösungsmittel und in einer Reaktionszeit von 0,1 bis 100 Stunden unter der Verwendung eines Katalysators, wie beispielsweise eines Pyridinderivates, wie typischerweise 4-(Dimethylamino)pyridine, oder eines Carbodiimidderivates wie typischerweise N,N'-Dicyclohexylcarbodiimide synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Außerdem werden die oligomeren oder polymeren Verbindungen der erfindungsgemäßen allgemeinen Formel I werden durch polymeranaloge Umsetzung einer 9,10-Bis(1,3-dithiol-2-yliden)-9,10-dihydroanthracenverbindung der allgemeinen Formel I'" mit einer oligomeren oder polymeren Verbindung der allgemeinen Formel P" synthetisiert. 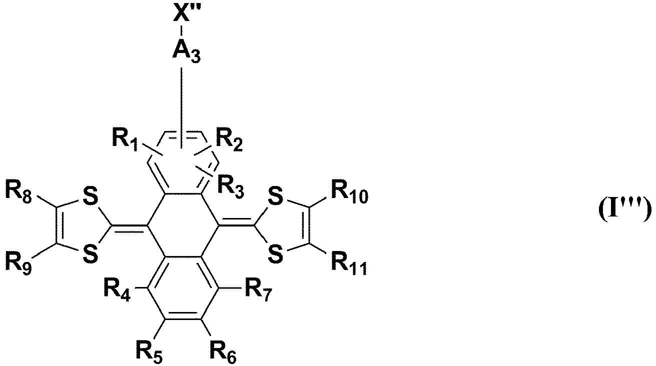 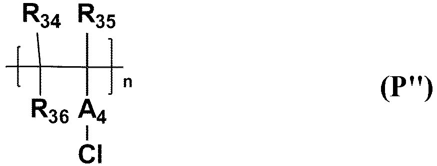 wobei
- R₁ bis R₇:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Halogenatome oder eine Kombination dieser Gruppen oder Atome darstellen, wobei Wasserstoffatome als mindestens fünf der Substituenten R₁ bis R₇ und Nicht-Wasserstoffatome, vorzugsweise Halogenatome, Alkylgruppen, Alkoxygruppen, Cyanogruppen und/oder Nitrogruppen, als null bis zwei der Substituenten R₁ bis R₇ und/oder Wasserstoffatome besonders bevorzugt sind,
- R₈ bis R₁₁:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei die Substituenten R₈ und R₉ bzw. die Substituenten R₁₀ und R₁₁ einen weiteren Ring bilden können, der aus fünf bis sieben Atomen besteht (der Ring kann aromatisch, heteroaromatisch, oder nicht aromatisch sein; ist der Ring nicht aromatisch, so kann er aus verschiedenen Gruppen, wie beispielsweise Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminoruppen, Monoalkylaminogruppen, Dialkylaminogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, bestehen, besonders bevorzugt entsprechen sich R₈ bis R₁₁ und sind Alkylgruppen, wie typischerweise Methylgruppen oder Ethylgruppen, Alkylthiogruppen, wie Methylthiogruppen, oder Ethylthiogruppen und Thiolgruppen),
- R₃₄ bis R₃₆:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Halogenatome oder eine Kombination dieser Gruppen oder Atome darstellen. Ganz besonders bevorzugt sind mindestens zwei der Substituenten R₃₄ bis R₃₆ Wasserstoffatome, und 0 bis 1 der Substituenten R₃₄ bis R₃₆ sind Nicht-Wasserstoffatome, vorzugsweise Halogenatome, Alkylgruppen, Alkoxygruppen, Cyanogruppen und/oder Nitrogruppen,
- X"':: eine nukleophile organische Gruppe ist, welche nukleophil an dem Nachbaratom des Halogenatom der Verbindung P" angreift und somit eine kovalente Bindung zwischen der Verbindung I"' und P" bildet, wobei eine Hydroxygruppe oder eine Thiolgruppe als X"' bevorzugt ist,
- A₃ und A₄:: eine kovalente Bindung, eine Alkylgruppe, eine Alkenylgruppe, eine Alkinylgruppe, eine Alkoxygruppe, eine Alkylthiogruppe, eine Cycloalkylgruppe, eine Cycloalkoxygruppe, eine Arylgruppe, eine Heteroarylgruppe, eine Aryloxygruppe, eine Aralkylgruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung oder eine Alkylgruppe als A₃ und A₄ besonders bevorzugt ist,
- n:: eine Ganzzahl größer gleich 2 ist.

Die Herstellung der Verbindung der allgemeinen Formel I mit Hilfe einer polymeranalogen Reaktion aus den aus den oben genannten Verbindungen I"' und P" ist in nachfolgenden Schemata 14-15 dargestellt, jedoch nicht auf diese beschränkt. 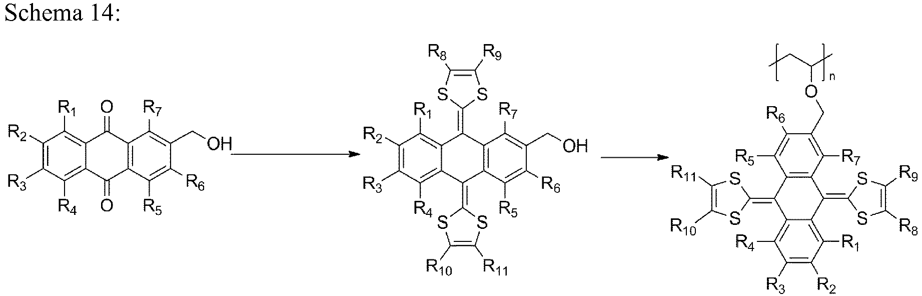 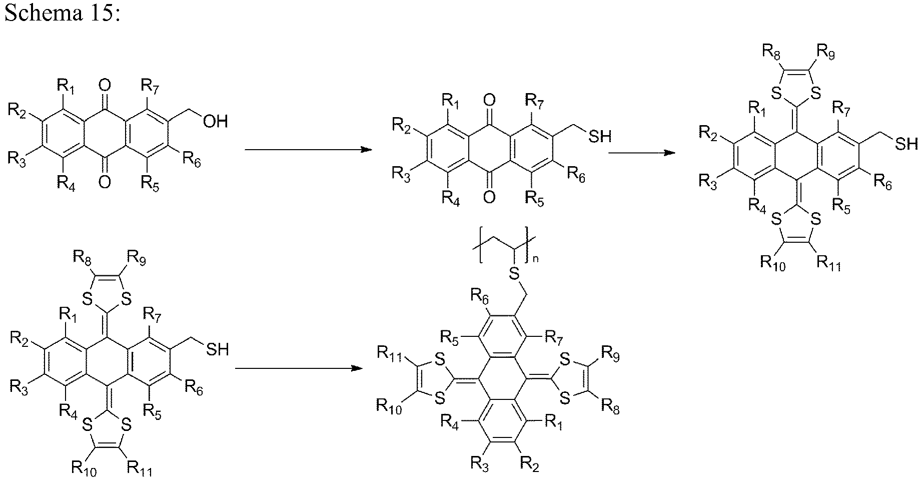 Für den Fall, dass X" in der allgemeinen Formel I'" einer Hydroxygruppe oder einer Thiolgruppe entspricht, kann die Verbindung der Formel I durch Reaktion der Verbindung I'" mit der Verbindung P" nach einer bekannten Methode der Synthese von Ethern und deren Abwandlungen synthetisiert werden. Bevorzugt wird die oben genannte Verbindung I in einem Temperaturbereich von -78 bis 150 °C, vorteilhaft in einem Temperaturbereich von - 40 bis 120 °C, in einem Lösungsmittel und in einer Reaktionszeit von 0,1 bis 100 Stunden unter der Verwendung eines Katalysators, wie beispielsweise Base wie Natriumhydrid, Natriumhydroxid, Kalium tert-Butylat, DBU, oder DBN synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Außerdem werden die oligomeren oder polymeren Verbindungen der erfindungsgemäßen allgemeinen Formel I durch polymeranaloge Umsetzung einer 9,10-Bis(1,3-dithiol-2-yliden)-9,10-dihydroanthracenverbindung der allgemeinen Formel I"" mit einer oligomeren oder polymeren Verbindung der allgemeinen Formel P'" synthetisiert. 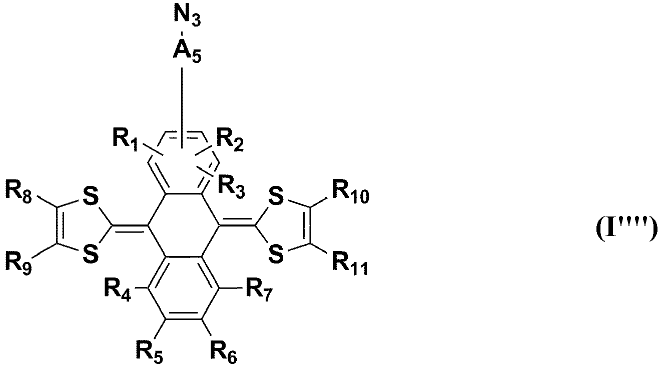 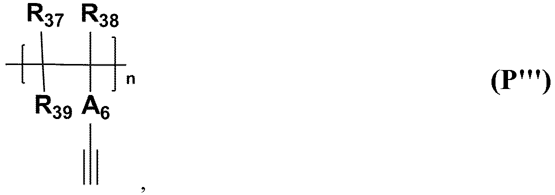 wobei
- R₁ bis R₇:: unabhängig voneinander Wasserstoffatome, Alkylgruppen, Alkenylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens fünf der Substituenten R₁ bis R₇ und Nicht-Wasserstoffatome, vorzugsweise Halogenatome, Alkylgruppen, Alkoxygruppen, Cyanogruppen und/oder Nitrogruppen, als null bis zwei der Substituenten R₁ bis R₇ besonders bevorzugt sind,
- R₈ bis R₁₁:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei die Substituenten R₈ und R₉ bzw. die Substituenten R₁₀ und R₁₁ einen weiteren Ring bilden können, der aus fünf bis sieben Atomen besteht (der Ring kann aromatisch, heteroaromatisch, oder nicht aromatisch sein; ist der Ring nicht aromatisch so kann er aus verschiedenen Gruppen, wie beispielsweise Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminoruppen, Monoalkylaminogruppen, Dialkylaminogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, bestehen, besonders bevorzugt entsprechen sich R₈ bis R₁₁ und sind Alkylgruppen, wie typischerweise Methylgruppen oder Ethylgruppen, Alkylthiogruppen, wie Methylthiogruppen, oder Ethylthiogruppen und Thiolgruppen),
- R₃₇ bis R₃₉:: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Halogenatome oder eine Kombination dieser Gruppen oder Atome darstellen. Ganz besonders bevorzugt sind mindestens zwei der Substituenten R₃₄ bis R₃₆ Wasserstoffatome, und 0 bis 1 der Substituenten R₃₄ bis R₃₆ sind Nicht-Wasserstoffatome, vorzugsweise Halogenatome, Alkylgruppen, Alkoxygruppen, Cyanogruppen und/oder Nitrogruppen,
- A₅ und A₆:: eine kovalente Bindung, eine Alkylgruppe, eine Alkenylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung, eine Arylgruppe oder eine Alkylgruppe als A₅ und A₆ besonders bevorzugt ist,
- n:: eine Ganzzahl größer gleich 2 ist.

Die Herstellung der Verbindung der allgemeinen Formel I mit Hilfe einer polymeranalogen Reaktion aus den aus den oben genannten Verbindungen I"" und P'" ist im nachfolgenden Schema 16 dargestellt, jedoch nicht auf dieses beschränkt.

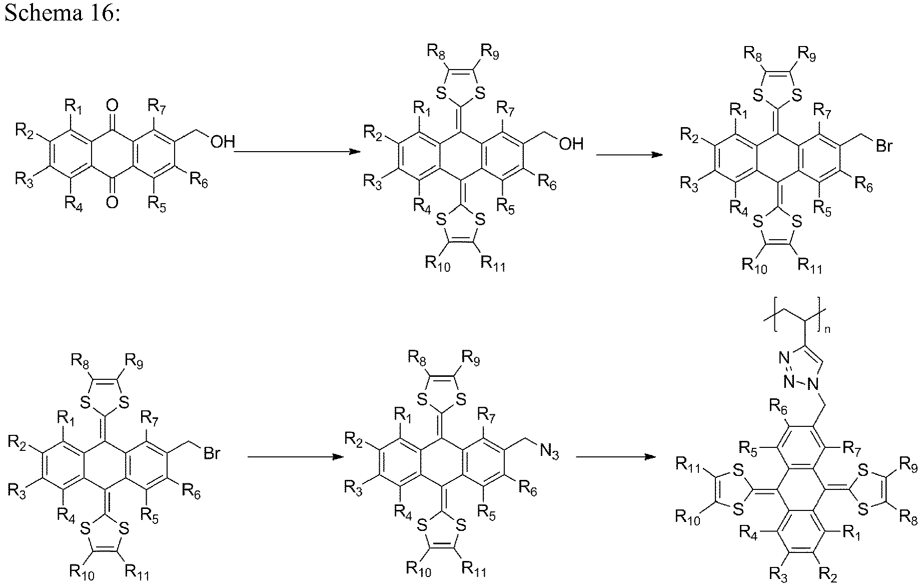

Die Verbindung der Formel I kann außerdem durch Reaktion der Verbindung I"" mit der Verbindung P'" nach einer bekannten Methode der Azid/Alkin Click-Reaktion und deren Abwandlungen synthetisiert werden. Bevorzugt wird die oben genannte Verbindung I in einem Temperaturbereich von -78 bis 150 °C, vorteilhaft in einem Temperaturbereich von - 40 bis 120 °C, in einem Lösungsmittel und in einer Reaktionszeit von 0,1 bis 100 Stunden synthetisiert. Für verwendete Lösungsmittel bestehen kaum Einschränkungen. Bevorzugt sind aprotische organische Lösungsmittel, wie beispielsweise N,N'-Dimethylformamid, N,N'-Dimethylacetamid, Dimethylsulfoxid, N-Methylpyrrolidin, Dichlormethan, 1,2-Dichlorethan, Toluol, Xylol, Chlorbenzol oder o-Dichlorbenzol.

Die oligomeren oder polymeren Verbindungen dieser Erfindung können zweckmäßig als redoxaktives Material zur Speicherung elektrischer Energie in einem elektrischen Ladungsspeicher verwendet werden.
Ein redoxaktives Material zur Speicherung elektrischer Energie ist ein Material, welches elektrische Ladung beispielsweise durch Aufnahme bzw. Abgabe von Elektronen speichern und wieder abgeben kann. Dieses Material kann beispielsweise als aktives Elektrodenmaterial in einem elektrischen Ladungsspeicher eingesetzt werden. Solche elektrischen Ladungsspeicher zur Speicherung elektrischer Energie sind z. B. Sekundärbatterien (Akkumulatoren), Redox-Flowbatterien und Superkondensatoren.

Eine sekundäre Batterie besteht aus einer negativen und einer positiven Elektrode, die durch einen Separator voneinander getrennt werden, sowie einem Elektrolyt, welche die Elektroden und den Separator umschließt.

Der Separator ist eine poröse Schicht, die den Ladungsausgleich zulässt, indem sie ionendurchlässig ist. Der Elektrolyt ist entweder ein Lösungsmittel, in dem ein Salz gelöst ist, oder eine oligomere oder polymere ionenleitende Verbindung. Die Hauptaufgabe des Elektrolyten ist die Ionenleitfähigkeit, die zum Ladungsausgleich nötig ist.

Eine Elektrode kann aus einer dünnen Schicht auf einem Substrat bestehen, welche sich aus einem Komposit, enthaltend zumindest ein Leitfähigkeitsadditiv, wenigstens ein Bindeadditiv sowie ein redoxaktives Material zur Ladungsspeicherung, welches die oligomere oder polymere Verbindung der erfindungsgemäßen allgemeinen Formel I sein kann, zusammensetzt. Dieses Komposit wird mit Hilfe eines Elektrodenslurrys auf einem Substrat aufgebracht.

Die besagte Schicht auf dem Substrat wird beispielsweise durch Anwendung einer bekannten Methode zur Filmbildung und deren Abwandlungen gebildet werden, bevorzugt durch verschiedene Druckverfahren, wie Offsetdruck, Siebdruck, Injektdruck, sowie durch eine Dipcoatingmethode, oder eine Aufschleudermethode, wobei die Schicht, welche die oligomere oder polymere Verbindung der erfindungsgemäßen allgemeinen Formel I beinhaltet, mit Hilfe eines Elektrodenslurry prozessiert wird. Dabei können die oligomere oder polymere Verbindung der Erfindung, das Leitfähigkeitsadditiv sowie das Bindeadditiv in einem Lösungsmittel suspendiert oder gelöst sein. Die Dicke der obengenannten Schicht, welche die oligomere oder polymere Verbindung dieser Erfindung enthält, ist nicht limitiert, beträgt aber bevorzugt zwischen 0,001 bis 5000 µm, besonders bevorzugt zwischen 0,01 und 1000 µm.

Als Substrat der oben genannten Elektrode werden Schichten von leitfähigen Materialien, bevorzugt Metalle, wie Platin, Gold, Eisen, Kupfer, Aluminium, Lithium oder eine Kombination aus diesen Metallen, sowie Kohlenstoffmaterialien, wie beispielsweise Glaskohlenstoff, Graphitfolie, Graphene oder Kohlenstofffelle, sowie Oxidsubstanzen, wie beispielsweise Indiumzinnoxid (ITO), Indiumzinkoxid (IZO), Antimonzinkoxid (AZO), Fluorzinnoxid (FTO) oder Antimonzinnoxid (ATO), verwendet.

Als Leitfähigkeitsadditiv für die Schicht finden unabhängig voneinander ein oder mehrere elektrisch leitende Materialien, bevorzugt Kohlenstoffmaterialen, wie beispielsweise Kohlenstofffasern, Kohlenstoffnanoröhren, Graphit, Ruß oder Graphene, sowie elektrisch leitende Polymere, wie beispielsweise Polyaniline, Polythiophene, Polyacetylene, PEDOT:PSS oder Polyarcene, Verwendung. Besonders bevorzugt werden Kohlenstofffasern verwendet.

Als Bindeadditive für das Substrat können unabhängig voneinander ein oder mehrere Materialien mit Bindeeigenschaften, bevorzugt Polymere, wie beispielsweise Polytetrafluoroethylen, Polyvinylidenefluorid, Polyhexafluoropropylen, Polyvinylchlorid, Polycarbonat, Polystyrol, Polyacrylate, Polymethacrylate, Polysulfone, Cellulosederivate, sowie Polyurethane eingesetzt werden.

Der Elektrodenslurry ist eine Lösung oder eine Suspension, bestehend aus beliebigen Anteilen an einem redoxaktiven Material zur Speicherung elektrischer Energie, wie beispielsweise die oligomere oder polymere Verbindung dieser Erfindung gemäß Formel I, einem Leitfähigkeitsadditiv und einem Bindeadditiv. Bevorzugt werden Anteile von 5 bis 100 Gewichtsprozent an einem redoxaktives Material zur Speicherung elektrischer Energie, 0 bis 80 Gewichtsprozent eines Leitfähigkeitsadditives und 0 bis 10 Gewichtsprozent eines Bindeadditives verwendet. Als Lösungsmittel für den Elektrodenslurry werden unabhängig voneinander ein oder mehrere Lösungsmittel, bevorzugt Lösungsmittel mit hohem Siedepunkt, wie beispielsweise N-Methyl-2-pyrrolidon, Wasser, Dimethylsulfoxid, Ethylencarbonat, Propylencarbonat, Dimethylcarbonat, Methylethylcarbonat, gamma-Butyrolacton, Tetrahydrofuran, Dioxolan, Sulfolan, N,N'-Dimethylformamid oder N,N'-Dimethylacetamid, verwendet. Die Konzentration des redoxaktiven Materials zur Speicherung elektrischer Energie im oben genannten Elektrodenslurry beträgt bevorzugt zwischen 0,1 und 10 mg/ml, besonders bevorzugt zwischen 0,5 und 5 mg/ml.

Die oligomeren oder polymeren Verbindungen dieser Erfindung gemäß allgemeiner Formel I können, je nach eingesetzter Gegenelektrode, als aktives Material zur elektrischen Ladungsspeicherung sowohl für die negative Elektrode oder für die positive Elektrode eingesetzt werden.

Wird die oligomere oder polymere Verbindung dieser Erfindung gemäß Formel I als redoxaktives Material zur elektrischen Ladungsspeicherung in der positiven Elektrode eingesetzt, so wird als redoxaktives Material zur elektrischen Ladungsspeicherung in der negativen Elektrode ein aktives Material verwendet, welches eine Redoxreaktion bei einem niedrigeren elektrochemischen Potential als die oligomere oder polymere Verbindung dieser Erfindung gemäß Formel I zeigt. Bevorzugt finden dabei Kohlenstoffmaterialien, wie beispielsweise Graphit, Graphene, Ruß, Kohlenstofffasern oder Kohlenstoffnanoröhren, sowie Metalle oder Legierungen, beispielsweise Lithium, Natrium, Magnesium, Lithium-Aluminium, Li-Si, Li-Sn, Li-Ti, Si, SiO, SiO₂, Si-SiO₂-Komplex, Zn, Sn, SnO, SnO₂, PbO, PbO₂, GeO, GeO₂, WO₂, MoO₂, Fe₂O₃, Nb₂O₅, TiO₂, Li₄Ti₅O₁₂, und Li₂Ti₃O₇, Anwendung.

Wird die oligomere oder polymere Verbindung dieser Erfindung gemäß Formel I als redoxaktives Material zur elektrischen Ladungsspeicherung in der negativen Elektrode eingesetzt, so wird als redoxaktives Material zur elektrischen Ladungsspeicherung in der positiven Elektrode, ein aktives Material verwendet, welches eine Redoxreaktion bei einem höherem elektrochemischen Potential als die oligomere oder polymere Verbindung dieser Erfindung gemäß Formel I zeigt. Bevorzugt wird dabei organisches redoxaktives Material zur elektrischen Ladungsspeicherung verwendet, wie beispielsweise eine oligomere oder polymere Verbindung mit einem stabilen organischen Radikal, eine oligomere oder polymere Verbindung mit einer Organoschwefeleinheit, eine oligomere oder polymere Verbindung mit einer Chinonstruktur, eine oligomere oder polymere Verbindung mit einem Dionsystem, eine oligomere oder polymere Verbindung mit einer Disulfidverbindung sowie eine oligomere oder polymere Verbindung mit einer Phenantrenstruktur und deren Derivate oder redoxaktives anorganisches Material zur Ladungsspeicherung, wie beispielsweise, LiCO₂, LiMn₂O₄, LiNiO₂, LiNi_{0,}5Mn_{0,5}O₂, LiFePO₄, LiMnO₄, LiCoPO₄, oder LiMnSiO₄, verwendet. Wird in der positiven Elektrode eine oben genannte redoxaktive oligomere oder polymere Verbindung eingesetzt, so kann diese Verbindung auch ein Komposit sein, bestehend aus dieser oligomeren oder polymeren Verbindung, einem Leitfähigkeitsadditiv und einem Bindeadditiv in einem beliebigen Verhältnis. Dieses Komposit kann wie oben beschrieben mit Hilfe eines Elektrodenslurrys durch ein bekanntes Verfahren zur Filmbildung als Schicht auf einem Substrat vorliegen.

Als redoxaktives Material zur Ladungsspeicherung kann auch Luft/Sauerstoff verwendet werden. In diesem Fall kann die positive Elektrode aus einem Leitfähigkeitsadditiv, einem Bindeadditiv und aus einem Redoxkatalysator bestehen. Bevorzugt werden als Redoxkatalysatoren ein anorganisches redoxaktives Material, wie beispielsweise Manganoxid, oder ein redoxaktives organisches Material, wie beispielsweise ein organisches Radikal, verwendet.

Als oben genannter Separator der besagten sekundären Batterie wird ein poröses Material, bevorzugt Membran bestehend aus einer polymeren Verbindung, wie beispielsweise Polyolefin, Polyamid, oder Polyester, verwendet. Die Aufgabe des Separators besteht darin, die positive Elektrode von der negativen Elektrode zu trennen und den Ladungsausgleich durch Permutation von Ionen zu ermöglichen.

Der oben genannte Elektrolyt der besagten Batterie kann sowohl eine Flüssigkeit, als auch eine oligomere oder polymere Verbindung mit hoher Ionenleitfähigkeit sein.
Ist der Elektrolyt flüssig, so setzt er sich unabhängig voneinander aus einem oder mehreren Lösungsmitteln und einem oder mehreren Leitsalzen zusammen.
Das Lösungsmittel der Elektrolyten besteht bevorzugt unabhängig voneinander aus einem oder mehreren Lösungsmitteln mit hohem Siedepunkt und hoher Ionenleitfähigkeit, aber niedriger Viskosität, wie beispielsweise Acetonitril, Dimethylsulfoxid, Ethylencarbonat, Propylencarbonat, Dimethylcarbonat, Diethylcarbonat, Methylethylcarbonat, gamma-Butyrolacton, Tetrahydrofuran, Dioxolan, 1,2-Dimethoxyethan, 1,2-Diethoxyethan, Digylme, Triglyme, Tetraglyme, Ethylacetat, 1,3-Dioxolan oder Wasser.

Das Leitsalz des Elektrolyten besteht aus einem Kation der Formel M^{e+} und einem Anion oder Formel An^{f-} der Formel (M^{e+})ₐ(An^{f-})_{b}, wobei e und f Ganzzahlen in Abhängigkeit der Ladung von M und An sind; a und b sind Ganzzahlen, welche die molekulare Zusammensetzung des Leitsalzes repräsentieren.

Als Kation des oben genannten Leitsalzes werden positiv geladene Ionen, bevorzugt Metalle der ersten und zweiten Hauptgruppe, wie beispielsweise Lithium, Natrium, Kalium oder Magnesium, aber auch andere Metalle der Nebengruppen, wie Zink, sowie organische Kationen, wie beispielsweise quartäre Ammoniumverbindungen wie Tetraalkylammoniumverbindungen, verwendet.

Als Anionen des besagten Leitsalzes werden bevorzugt anorganische Anionen, wie Hexafluorophosphat, Tetrafluoroborat, Triflat, Hexafluoroarsenat, Hexafluoroantimonat, Tetrafluoroaluminat, Tetrafluoroindat, Perchlorat, Bis(oxolato)borat, Tetrachloroaluminiat, Tetrachlorogallat, aber auch organische Anionen, wie beispielsweise N(CF₃SO₂)₂⁻, CF₃SO₃⁻, Alkoholate, wie beispielsweise, tert-Butanolat, oder i-Propylalkoholat aber auch Halogenide, wie Fluorid, Chlorid, Bromid sowie Iodid, verwendet.

Die Erfindung soll nachstehend anhand von in der Zeichnung dargestellten Ausführungsbeispielen zur Herstellung und Verwendung näher erläutert werden.
Es zeigen:
- Abb. 1:: Cyclovoltammogramm (14 Zyklen) einer gemäß Beispiel 4 hergestellten Elektrode.
- Abb. 2:: Lade/Entladekurven des ersten und des zweihundertfünfzigsten Lade/Entladezyklus einer sekundären Batterie, hergestellt gemäß Beispiel 5.
- Abb. 3:: Lade/Entladeverhalten der sekundären Batterie, hergestellt gemäß Beispiel 5.
- Abb. 4:: Cyclovoltammogramm (20 Zyklen) einer gemäß Beispiel 9 hergestellten Elektrode.
- Abb. 5:: Lade/Entlade-Verhalten der sekundären Batterie, hergestellt gemäß Beispiel 10
- Abb. 6:: Lade/Entlade-Verhalten der sekundären Zink-organischen-Batterie bei unterschiedlichen Ladegeschwindigkeiten (10 C = volles Laden in 6 Minuten; 20 C = volles Laden in 3 Minuten; 40 C = volles Laden in 1 Minute, 30 Sekunden; 60 C = volles Laden in 1 Minute; 90 C = volles Laden in 45 Sekunden; 120 C = volles Laden in 30 Sekunden), hergestellt gemäß Beispiel 10.

¹H and ¹³C NMR Spektren wurden mit einem Bruker AC 300 (300 MHz) Spectrometer bei 298 K aufgenommen. Elementaranalysen wurden mit einem Vario ELIII-Elementar Euro Gerät sowie einem EA-HekaTech Gerät durchgeführt. Für Cyclovoltammetrie und galvinostatische Experimente stand ein Biologic VMP 3 Potentiostant zur Verfügung. Größenausschlusschromatorgraphie wurde an einem Agilent 1200 series System (Entgaser: PSS, Pumpe: G1310A, Autosampler: G1329A, Oven: Techlab, DAD Detector: G1315D, RI Detector: G1362A, Eluent: DMAc + 0.21% LiCl, 1 ml/min, Temperatur: 40 °C, Säule: PSS GRAM guard/1000/30 Å) durchgeführt.

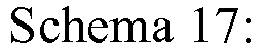

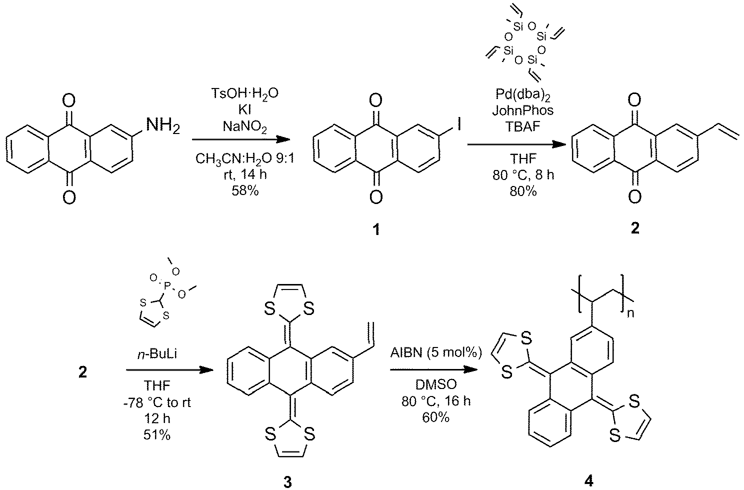

### Beispiel 1:

### Synthese von 2-Vinylanthrachinon (2 in vorstehendem Schema 17):

2-Iodanthrachinon (1.74 g, 5.22 mmol), Bis(dibenzylidenaceton)palladium(0) (0.060 g, 0.104 mmol) und Biphenyl-2-yldi-tert-butylphosphin (0.062 g, 0.209 mmol) werden in einer 0.3 M Lösung von Tetrabutylammoniumfluorid in Tetrahydrofuran gelöst. Die Lösung wird mit Argon gespült und 2,4,6,8-Tetramethyl-2,4,6,8-tetravinyl-1,3,5,7,2,4,6,8-tetraoxatetrasilocan (0.902 ml, 2.61 mmol) wird tropfenweise hinzugegeben. Die Mischung wird acht Stunden unter Argonatmosphäre bei 80°C gerührt. Die Reaktionsmischung wird auf Raumtemperatur abgekühlt und in 250 ml Ethanol gegeben. Der Niederschlag wird abfiltriert und zweimal mit n-Hexan gewaschen. Nach der Trocknung im Vakuum wird fast reines 2-Vinylanthrachinon (**2**) (1.175 g, 5.02 mmol, 96%) als gelblicher Feststoff, dessen Reinheit für den nächsten Reaktionsschritt ausreichend ist, erhalten.
Anal. Calcd for C₁₆H₁₀O₂: C, 81.90; H, 4.30. Found: C, 81.85; H, 4.31. ¹H NMR (CDCl₃, 300 MHz, ppm): δ 5.54 (d, 1H), 6.05 (d, 1H), 6.87 (dd, 1H), 7.80 (m, 3H), 8.32 (m, 4H). ¹³C NMR (CDCl₃, 75 MHz, ppm): δ 183.2, 182.6, 143.2, 135.4, 134.1, 134.0, 133.8, 133.6, 133.5, 132.5,131.4, 128.3, 127.8, 127.2, 124.8, 118.4.

### Beispiel 2:

### Synthese von 2,2'-(2-Vinylanthracen-9,10-diyliden)bis(1,3-dithiol) (3 in Schema 17):

Dimethyl-1,3-dithiol-2-ylphosphonat (733 mg, 3.45 mmol) wird in 10 ml Tetrahydrofuran unter Argon Atmosphäre gelöst und die Reaktionsmischung wird auf -78°C abgekühlt. Eine 2.5 M Lösung von n-Buthyllithium in n-Hexan (1.5 ml, 3.75 mmol) wird tropfenweise zur Reaktionsmischung innerhalb von fünf Minuten hinzugefügt. Die Reaktionsmischung wird zwei Stunden bei -78°C gerührt. Danach wird eine Lösung aus 2-Vinylanthrachinon (352 mg, 1.50 mmol) in 11.5 ml Tetrahydrofuran bei -78°C tropfenweise hinzugegeben. Nach einer Stunde bei -78 °C wird die Reaktionsmischung weitere vier Stunden bei Raumtemperatur gerührt. Zur Reaktionsmischung werden 50 ml Ethylacetat gegeben und die Mischung wird zweimal mit Wasser (35 ml) und einmal mit Brine (20 ml) extrahiert. Die organische Phase wird über Magnesiumsulfat getrocknet, filtriert und bei reduziertem Druck aufkonzentriert. Das erhaltene Rohprodukt wird mittels Säulenchromatographie aufgereinigt (Kieselgel n-Hexan/Toluol, 1/1). Dabei werden 700 mg (2.12 mmol, 51%) (**3**) als gelbes Pulver erhalten. Anal. Calcd for C₂₂H₁₀N₄: C, 64.99; H, 3.47, S, 31.54. Found: C, 64.81; H, 3.58, S 30.95. 1H NMR (CD₂Cl₂, 300 MHz, ppm): δ 7.78 (d, 1H), δ 7.74-7.69 (m, 2H), δ 7.67 (s, 1H), δ 7.38 (d, 1H), δ 7.36 (m, 2H), δ 6.81 (dd, 1H), δ 6.39 (s, 2H), δ 6.38 (s, 1H), δ 5.84 (d, 1H), δ 5,33 (d, 1H). ESI-MS, m/z 406.00 [M+].

### Beispiel 3:

### Synthese von Poly(2,2'-(2-vinylanthracen-9,10-diyliden)bis(1,3-dithiol)) (4 in Schema 17):

50 mg 2,2'-(2-vinylanthracen-9,10-diyliden)bis(1,3-dihiol) (**3**) werden in 0.25 ml Dimehylsulfoxid gelöst und mit 1.01 mg AIBN (0.0062 mmol, 5 mol%) versetzt. Die Reaktionsmischung wird fünf Minuten lang mit Argon entgast und 18 Stunden bei 80°C gerührt. Danach wird die Reaktionslösung in 50 ml Dichlormethan gegeben, um das Produkt auszufällen. Dabei entstehen 30 mg Poly2,2'-(2-vinylanthracen-9,10-diyliden) bis(1,3-dihiol) (**4**) als oranger Feststoff.
Anal. Calcd for C₂₂H₁₀N₄: C, 80.00; H, 3.10, N, 16.90. Found: C, 79.96; H, 3.13, N 16.95. ¹H NMR (DMF-d₇, 300 MHz, ppm): δ 8.83 to 7.48 (br, 7H), 2.62 to 1.31 (br, 3H). SEC: Mₙ 6.02x10³ g/mol (PS-Standard), PDI: 1.66.

### Beispiel 4:

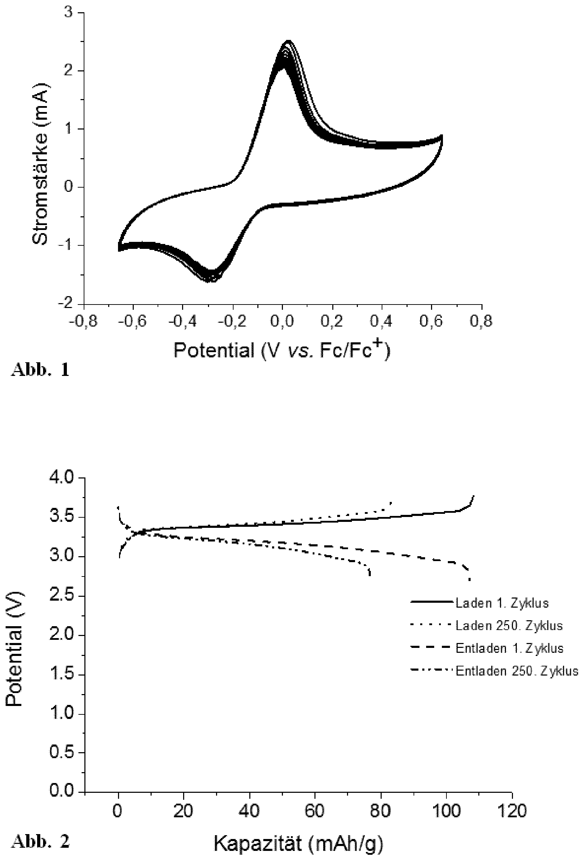

### Herstellung einer Elektrode mit Poly(2,2'-(2-vinylanthracen-9,10-diyliden)bis(1,3-dithiol)) (4 in Schema 17), vgl. Abb. 1:

Eine Lösung bestehend aus Poly(2,2'-(2-vinylanthracen-9,10-diyliden)bis(1,3-dithiol)) (**4**) in NMP (N-Methyl-2-pyrrolidon) (10 mg/ml) wurde zu Kohlenstofffasern (VGCF; Showa-Denko) als Leitaddiv und Poly(vinylidenefluorid) (PVDF; Sigma Aldrich als Bindeadditiv (Verhältnis: 10/80/10 v/m/m) hinzugefügt. Diese Materialien wurden in einem Mörser zehn Minuten lang durchmischt und die dabei erhaltene Paste wurde unter Anwendung einer Streichmessermethode auf eine Aluminiumfolie (Dicke: 0.015 mm, MTI Corporation) aufgebracht. Die Elektrode wird bei 100°C 24 Stunden lang getrocknet.

Die Elektrode wird in eine Elektrolytlösung (0,1 M LiClO₄ in 1,2-Dimethoxyethan/ Propylencarbonat 4/1) eingetaucht. Für die cyclovoltammographische Messung wird eine Halbzelle, bestehend aus der besagten Elektrode als Arbeitselektrode und einer Ag/AgNO₃-Elektrode als Referenzelektrode sowie einem Platinnetz als Gegenelektrode, aufgebaut (Abb. 1).

Das Cyclovoltammogramm zeigt eine stabile Redoxreaktion bei -0.12 V.

### Beispiel 5:

### Herstellung einer Li-Polymerbatterie:

Die in Beispiel 4 beschriebene Elektrode wird unter Argonatmosphäre in eine sekundäre Batterie (Li-Polymerbatterie) eingebracht. Als Elektrolyt dient eine 0.1 M Lösung aus LiClO₄ in 1,2-Dimethoxyethan/Propylencarbonat 4/1, als Gegenelektrode dient ein Stück elemtares Lithium. Die beiden Elektroden werden durch den Separator (eine poröse Polypropylenmembran, Celgard) voneinander getrennt. Die Batterie zeigt ein Ladeplateau bei 3.4 V und ein Entladeplateau bei 3.2 V (Abb. 2).

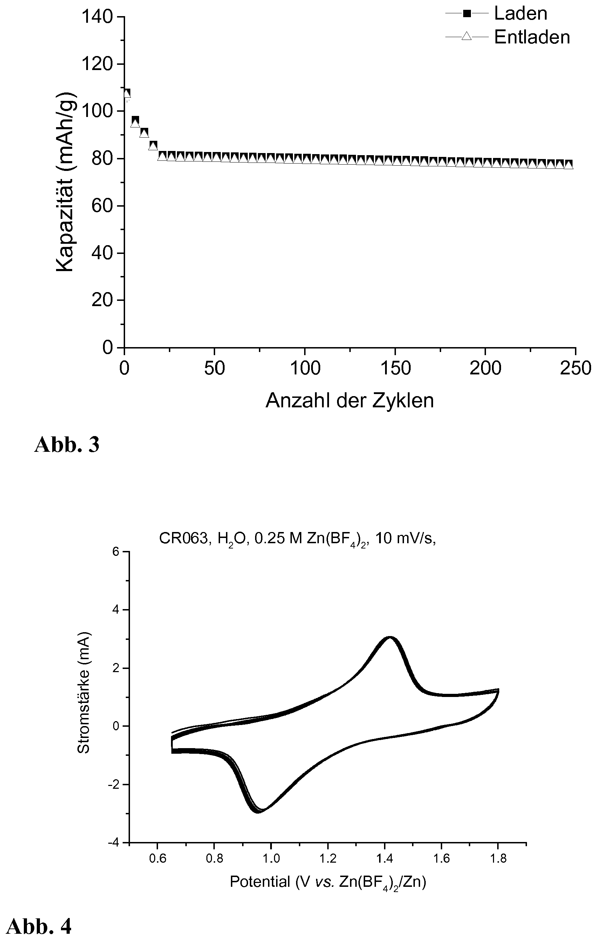

Im ersten Lade/Entladezyklus zeigt die Batterie eine Kapazität von 108 mAh/g (82% der theoretisch möglichen Kapazität), nach 500 Lade/Entladezyklen zeigt die Batterie eine Kapazität von 82 mAh/g (Abb. 3) bei einer durchschnittlichen Coulombeffizienz von 99%.

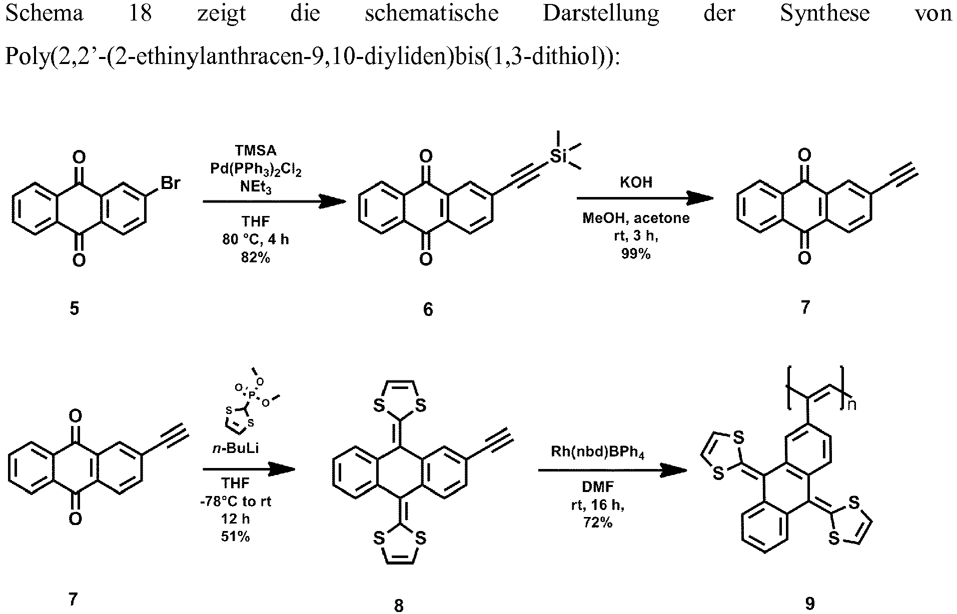

### Beispiel 6:

### Synthese von 2-Ethinylanthrachinon (7 in Schema 18):

2-Bromanthrachinon (**5** in Schema 18; 1,00 g, 3,5 mmol), Kupfer(I)iodid (0,012 mg, 0,007 mmol) und Bis(triphenylphosphin)palladium(II)dichlorid (0.042 g, 0.035 mmol) werden in einer Mischung aus Tetrahydrofuran und Triethylamin 1/1 v/v (11 mL) gelöst. Die Lösung wird mit Argon gespült und Trimethylsilylacetlyen (0.54 ml, 3,8 mmol) wird tropfenweise hinzugegeben. Die Mischung wird sechs Stunden unter Argonatmosphäre bei 80°C gerührt. Die Reaktionsmischung wird auf Raumtemperatur abgekühlt und 50 ml Chloroform werden hinzugegeben. Die Lösung wird je einmal mit 50 ml gesättiger wässriger Ammoniumchlorid Lösung, einmal mit 50 ml Wasser und einmal mit 50 ml gesättigter wässriger Natriumchlorid Lösung gewaschen, über Magnesiumsulfat getrocknet, filtriert und unter vermindertem Druck bis zur Trockne eingeengt. Das Rohprodukt wird mittels Säulenchromatographie (Chloroform/ n-Hexan 5/1) aufgereinigt. Das erhaltene 2-((Trimethylsilyl)ethinyl)anthrachinon (800 mg, 2,64 mmol) wird in 25 ml einer Aceton/Methanol Lösung im Verhältnis von 2/1 gelöst. Zu dieser Lösung wird Natriumhydroxid (105,2 mg, 2,64 mmol) zugegeben. Die Reaktionsmischung wird drei Stunden bei Raumtemperatur gerührt, mit 50 ml Chloroform verdünnt und mit einmal 50 ml gesättiger wässriger Ammoniumchlorid Lösung, einmal mit 50 ml Wasser und einmal mit 50 ml gesättigter wässriger Natriumchlorid Lösung gewaschen, über Magnesiumsulfat getrocknet, filtriert und unter vermindertem Druck bis zur Trockne eingeengt. Das Rohprodukt wird mittels Säulenchromatographie (Chloroform/ n-Hexan 5/1) auf gereinigt. Es wurden 682 mg 2-Ethinylanthrachinon (**7** in Schema 18) als braune Kristalle erhalten.
Anal. calcd for C₁₆H₈O₂: C, 82.8; H, 3.5 %. Found: C, 82.7; H, 3.3 %. ¹H NMR (CDCl₃, 300 MHz, ppm, TMS): d 8.32 (Ph, 4H), 7.83 (Ph, 3H), 3.37 (CH, 1H). ¹³C NMR (CDCl₃, 300 MHz, ppm, TMS): d 182.8, 137.5, 134.8, 134.7, 133.9, 133.8, 133.3, 131.3, 128.7, 127.8, 127.5, 82.4, 82.3. MS (m/z) calcd for M⁺ 232.1. Found: 233.1.

### Beispiel 7:

### Synthese von 2,2'-(2-Ethinylanthracen-9,10-diyliden)bis(1,3-dithiol) (8 in Schema 18)

Dimethyl-1,3-dithiol-2-ylphosphonat (823 mg, 3,2 mmol) wird in 5 ml Tetrahydrofuran unter Argon Atmosphäre gelöst und die Reaktionsmischung wird auf -78°C abgekühlt. Eine 2.5 M Lösung von n-Buthyllithium in n-Hexan (1,5 ml, 3.75 mmol) wird tropfenweise zur Reaktionsmischung innerhalb von fünf Minuten hinzugefügt. Die Reaktionsmischung wird zwei Stunden bei -78°C gerührt. Danach wird eine Lösung aus 2-Ethinylanthrachinon (**7** in Schema 18; 352 mg, 1.50 mmol) in 11.5 ml Tetrahydrofuran bei -78°C tropfenweise hinzugegeben. Nach einer Stunde bei -78 °C wird die Reaktionsmischung weitere vier Stunden bei Raumtemperatur gerührt. Zur Reaktionsmischung werden 50 ml Chloroform, gegeben und die Mischung wird zweimal mit Wasser (35 ml) und einmal mit gesättigter wässriger Kochsalzlösung (20 ml) extrahiert. Die organische Phase wird über Magnesiumsulfat getrocknet, filtriert und bei reduziertem Druck aufkonzentriert. Das erhaltene Rohprodukt wird mittels Säulenchromatographie aufgereinigt (Kieselgel n-Hexan/Toluol, 1/1). Dabei werden 418 mg (1.03 mmol, 69%) Produkt (**8** in Schema 18) als gelber Feststoff erhalten. Anal. Calcd for C₂₂H₁₂S₄: C, 65.31; H, 2.99, S, 31.70. Found: C, 65.20; H, 2.90, S 31.62.

### Beispiel 8:

### Synthese von Poly(2,2'-(2-ethinylanthracen-9,10-diyliden)bis(1,3-dithiol)) (9 in Schema 18)

Unter Argon wird eine Lösung von 50 mg 2,2'-(2-Ethinylanthracen-9,10-diyliden)bis(1,3-dihiol) (**8** in Schema 18) in 0.25 ml *N,N*-Dimethylformamid mit einer Lösung von 1.49 mg Bicyclo[2.2.1]hepta-2,5-diene-rhodium(I) chlorid dimer in 0,1 ml *N,N*-Dimethylformamid versetzt. Die Reaktionslösung wird 18 Stunden bei Raumtemperatur gerührt. Danach wird die Reaktionslösung in 50 ml Acetonitril gegeben, um das Produkt auszufällen. Dabei entstehen 40 mg Poly2,2'-(2-ethinylanthracen-9,10-diyliden) bis(1,3-dithiol) (**9** in Schema 18) als oranger Feststoff.
Anal. Calcd for C₂₂H₁₂S₄: C, 65.31; H, 2.99, S, 31.70. Found: C, 65.29; H, 2.80, S 31.52. SEC: Mₙ 8.92x10³ g/mol (PS-Standard), PDI: 1.84.

### Beispiel 9:

### Herstellung einer Elektrode mit Poly(2,2'-(2-ethinylanthracen-9,10-diyliden)bis(1,3-dithiol))

Eine Lösung bestehend aus Poly(2,2'-(2-ethinylanthracen-9,10-diyliden)bis(1,3-dithiol)) (**9** in Schema 18) in NMP (N-Methyl-2-pyrrolidon) (5 mg/ml) wurde zu Kohlenstofffasern (MWCNT; Sigma-Aldirch) als Leitaddiv (Verhältnis: Polymer/Leitadditive 50/50 m/m) hinzugefügt. Diese Materialien wurden in einem Mörser zehn Minuten lang durchmischt und die dabei erhaltene Paste wurde unter Anwendung einer Streichmessermethode auf eine Graphitfolie (Dicke: 0.254 mm, Alfa Aesar) aufgebracht. Die Elektrode wird bei 100°C 24 Stunden lang getrocknet.

Die Elektrode wird in eine Elektrolytlösung (2 M Zn(BF₄)₂ in Wasser) eingetaucht. Für die cyclovoltammographische Messung wird eine Halbzelle, bestehend aus der besagten Elektrode als Arbeitselektrode und einer Ag/AgCl-Elektrode als Referenzelektrode sowie einer Zinkfolie als Gegenelektrode, aufgebaut

Das Cyclovoltammogramm (Abbildung 4) zeigt eine stabile Redoxreaktion zwischen 0,8 und 1,6 V.

### Beispiel 10:

### Herstellung einer Zink-Polymerbatterie:

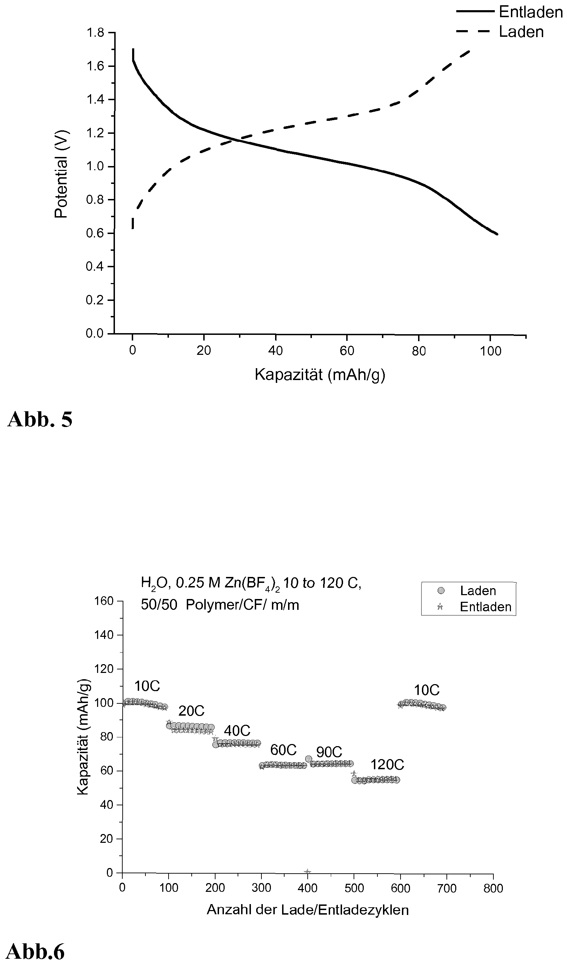

Die in Beispiel 9 beschriebene Elektrode wird in eine sekundäre Batterie (Zn-Polymerbatterie) eingebracht. Hierbei fungiert Zinkmetall als Anode und die Polymerkompositeelektrode als Kathode. Als Elektrolyt dient eine 2 M Lösung aus Zn(BF₄)₂ in Wasser, als Gegenelektrode dient ein Stück elementare Zinkfolie. Die beiden Elektroden werden durch den Elektrolyten (Abstand ca. 3 mm) voneinander getrennt. Die Batterie zeigt ein Ladeplateau bei 1.2 V und ein Entladeplateau bei 1.1 V (Abb. 5).

Im ersten Lade/Entladezyklus bei einer Geschwindigkeit von 10C (= volles Laden in 6 Minuten) zeigt die Batterie eine Kapazität von 100 mAh/g (78% der theoretisch möglichen Kapazität), nach 100 Lade/Entladezyklen zeigt die Batterie eine Kapazität von 95 mAh/g (Abb. 6) bei einer durchschnittlichen Coulombeffizienz von 99% (Coulombeffizienz = Verhältnis der während eines Lade/Entladezyklus der Batterie entnommenen Ladung zu der während desselben Lade/Entladezyklus der Batterie zugeführten Ladung). Die Batterie kann mit bis zu 120C geladen werden. Bei einer Ladegeschwindigkeit von 120C (volles Laden in 30 Sekunden) zeigt die Batterie eine Kapazität von 55 mAh/g (40% Materialaktivität).

## Patentansprüche

1. 9,10-Bis(1,3-dithiol-2-yliden)-9,10-dihydroanthracenpolymere, bestehend aus einer oligomeren oder polymeren Verbindung der allgemeinen Formel I, 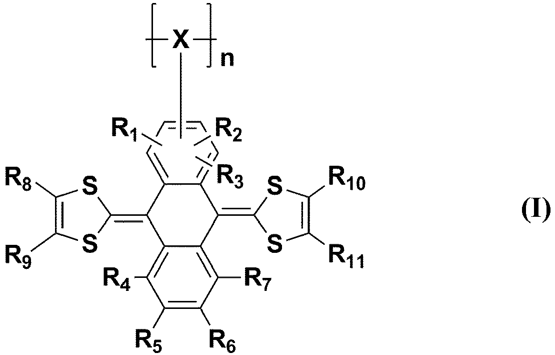 wobei
R₁ bis R₇: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens fünf der Substituenten R₁ bis R₇ und Nicht-Wasserstoffatome, vorzugsweise Halogenatome, Alkylgruppen, Alkoxygruppen, Cyanogruppen und/oder Nitrogruppen, als null bis zwei der Substituenten R₁ bis R₇ besonders bevorzugt sind,
R₈ bis R₁₁: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei die Substituenten R₈ und R₉ bzw. die Substituenten R₁₀ und R₁₁ einen weiteren Ring bilden können, der aus fünf bis sieben Atomen besteht (der Ring kann aromatisch, heteroaromatisch, oder nicht aromatisch sein; ist der Ring nicht aromatisch, so kann er aus verschiedenen Gruppen, wie beispielsweise Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Aminoruppen, Monoalkylaminogruppen, Dialkylaminogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, bestehen, besonders bevorzugt entsprechen sich R₈ bis R₁₁ und sind Alkylgruppen, wie typischerweise Methylgruppen oder Ethylgruppen, Alkylthiogruppen, wie Methylthiogruppen, oder Ethylthiogruppen und Thiolgruppen),
X: eine organische Gruppe ist, welche durch Polymerisationreaktion aus einer Gruppe gebildet wird, die aus einer organischen Doppelbindung, einer organischen Dreifachbindung, eines Oxirans oder eines Aziridins besteht, oder eine organische Gruppe ist, welche durch eine polymeranaloge Reaktion gebildet wird,
n: eine Ganzzahl größer gleich 2 ist.

2. 9,10-Bis(1,3-dithiol-2-yliden)-9,10-dihydroanthracenpolymere gemäß Anspruch 1 **dadurch gekennzeichnet, dass** X eine organische Gruppe einer der allgemeinen Formeln II - XIV darstellt. 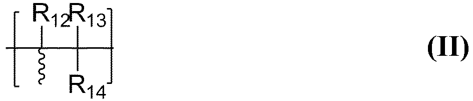 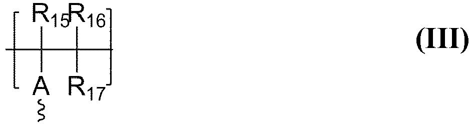 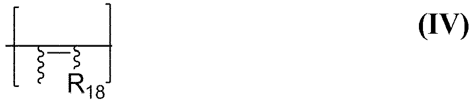 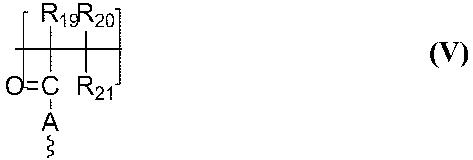 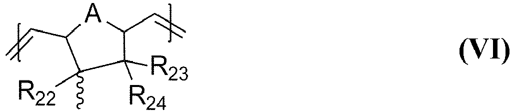 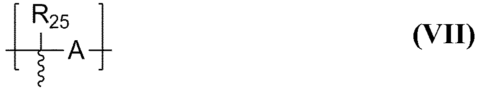 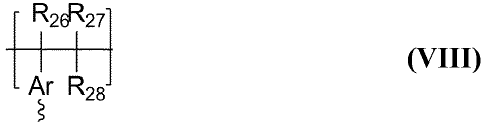 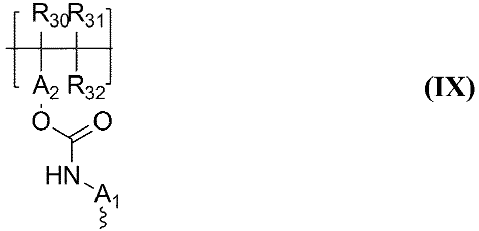 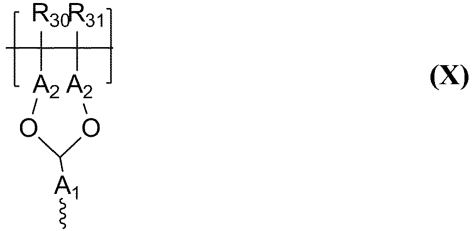 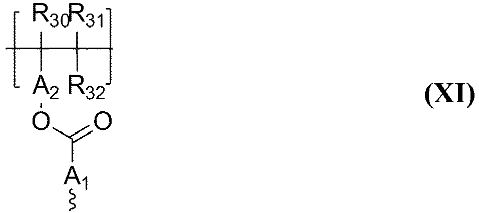 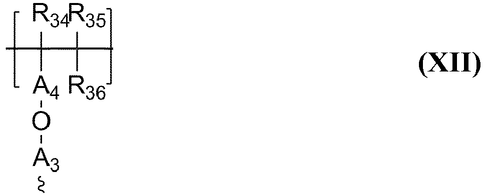 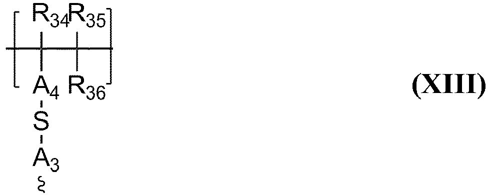 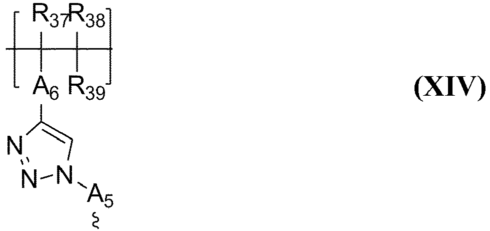 wobei
R₁₂ bis R₂₈: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei
Wasserstoffatome als mindestens zwei der Substituenten R₁₂ bis R₁₄ und Nicht-Wasserstoffatome, vorzugsweise Halogenatome, Alkylgruppen, Alkoxygruppen, Cyanogruppen und/oder Nitrogruppen, als null bis zwei der Substituenten R₁₂ bis R₁₄ und/oder
Wasserstoffatome als mindestens zwei der Substituenten R₁₅ bis R₁₇ und Nicht-Wasserstoffatome, vorzugsweise Halogenatome, Alkylgruppen, Alkoxygruppen, Cyanogruppen und/oder Nitrogruppen, als null bis einem der Substituenten R₁₅ bis R₁₇ und/oder
ein Wasserstoffatom als R₁₈, und/oder
Wasserstoffatome als mindestens zwei der Substituenten R₁₉ bis R₂₁ und Nicht-Wasserstoffatome, vorzugsweise Halogenatome, Alkylgruppen, Alkoxygruppen, Cyanogruppen und/oder Nitrogruppen, als null bis einem der Substituenten R₁₉ bis R₂₁, und/oder
Wasserstoffatome als mindestens zwei der Substituenten R₂₂ bis R₂₄ und Nicht-Wasserstoffatome, vorzugsweise Halogenatome, Alkylgruppen, Alkoxygruppen, Cyanogruppen und/oder Nitrogruppen, als null bis einem der Substituenten R₂₂ bis R₂₄ und/oder
ein Wasserstoffatom als R₂₅ und/oder
Wasserstoffatome als mindestens zwei der Substituenten R₂₆ bis R₂₈ und Nicht-Wasserstoffatome, vorzugsweise Halogenatome, Alkylgruppen, Alkoxygruppen, Cyanogruppen und/oder
Nitrogruppen, als null bis einem der Substituenten R₂₆ bis R₂₈ besonders bevorzugt sind,
R₃₀ bis R₃₂: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens zwei der Substituenten R₃₀ bis R₃₂ und Nicht-Wasserstoffatome, vorzugsweise Halogenatome, Alkylgruppen, Alkoxygruppen, Cyanogruppen und/oder Nitrogruppen, als null bis einem der Substituenten R₃₀ bis R₃₂ besonders bevorzugt sind,
R₃₄ bis R₃₆: unabhängig voneinander Wasserstoffatome, Alkylgruppen, Alkenylgruppen, Alkinylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Halogenatome oder eine Kombination dieser Gruppen oder Atome darstellen, wobei Wasserstoffatome als mindestens zwei der Substituenten R₃₄ bis R₃₆, und Nicht-Wasserstoffatome, vorzugsweise Halogenatome, Alkylgruppen, Alkoxygruppen, Cyanogruppen und/oder Nitrogruppen, als null bis einem der Substituenten R₃₄ bis R₃₆ besonders bevorzugt sind,
R₃₇ bis R₃₉: unabhängig voneinander Wasserstoffatome, Alkylgruppen, Alkenylgruppen, Alkoxygruppen, Alkylthiogruppen, Haloalkylgruppen, Haloalkoxygruppen, Cycloalkylgruppen, Cycloalkoxygruppen, Arylgruppen, Heteroarylgruppen, Aryloxygruppen, Aralkylgruppen, Carbonsäuregruppen, Sulfonsäuregruppen, Aminogruppen, Monoalkylaminogruppen, Dialkylaminogruppen, Nitrogruppen, Cyanogruppen, Hydroxylgruppen, Alkylcarbonylgruppen, Alkenylcarbonylgruppen, Alkinylcarbonylgruppen, Carbonsäureestergruppen, Carbonsäureamidgruppen, Sulfonsäureestergruppen, Thiolgruppen, Halogenatome oder eine Kombination dieser Gruppen oder Atome darstellen, wobei Wasserstoffatome als mindestens zwei der Substituenten R₃₇ bis R₃₉ und Nicht-Wasserstoffatome, vorzugsweise Halogenatome, Alkylgruppen, Alkoxygruppen, Cyanogruppen und/oder Nitrogruppen, als null bis einem der Substituenten R₃₇ bis R₃₉ besonders bevorzugt sind,
A: ein Sauerstoffatom, ein Schwefelatom oder eine -N(R₃₃)- Gruppe ist, wobei R₃₃ ein Wasserstoffatom, eine Alkylgruppe, eine Alkenylgruppe, eine Alkinylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Carbonsäuregruppe, eine Sulfonsäuregruppe, eine Nitrogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe ist, wobei ein Sauerstoffatom als A besonders bevorzugt ist,
A₁ und A₂: unabhängig voneinander eine kovalente Bindung, eine Alkylgruppe, eine Alkenylgruppe, eine Alkinylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung oder eine Alkylgruppe als A₁ und A₂ besonders bevorzugt ist,
A₃ und A₄: unabhängig voneinander eine kovalente Bindung, eine Alkylgruppe, eine Alkenylgruppe, eine Alkinylgruppe, eine Alkoxygruppe, eine Alkylthiogruppe, eine Cycloalkylgruppe, eine Cycloalkoxygruppe, eine Arylgruppe, eine Heteroarylgruppe, eine Aryloxygruppe, eine Aralkylgruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung oder eine Alkylgruppe als A₃ und A₄ besonders bevorzugt ist,
A₅ und A₆: unabhängig voneinander eine kovalente Bindung, eine Alkylgruppe, eine Alkenylgruppe, eine Alkoxygruppe, eine Alkylthiogruppe, eine Haloalkylgruppe, eine Haloalkoxygruppe, eine Cycloalkylgruppe, eine Cycloalkoxygruppe, eine Arylgruppe, eine Heteroarylgruppe, eine Aryloxygruppe, eine Aralkylgruppe, eine Monoalkylaminogruppe, eine Dialkylaminogruppe, eine Alkylcarbonylgruppe, eine Alkenylcarbonylgruppe, eine Alkinylcarbonylgruppe, eine Carbonsäureestergruppe, eine Carbonsäureamidgruppe, eine Sulfonsäureestergruppe sind, wobei eine kovalente Bindung, eine Arylgruppe oder eine Alkylgruppe als A₅ und A₆ besonders bevorzugt ist,
Ar: einer unabhängig voneinander substituierten Cycloalkylgruppe, Cycloalkoxygruppe, Arylgruppe, Heteroarylgruppe, Aryloxygruppe, Aralkylgruppe entspricht.

3. Verwendung der 9,10-Bis(1,3-dithiol-2-yliden)-9,10-dihydroanthracenpolymere gemäß Anspruch 1 oder 2 als aktives Elektrodenmaterial für elektrische Ladungsspeicher, insbesondere Sekundärbatterien.

4. Verwendung der 9,10-Bis(1,3-dithiol-2-yliden)-9,10-dihydroanthracenpolymere gemäß Anspruch 3, **dadurch gekennzeichnet, dass** das aktive Elektrodenmaterial als vollständige oder teilweise Oberflächenbeschichtung von Elektrodenelementen der elektrischen Ladungsspeicher, insbesondere Sekundärbatterien, ausgebildet ist.

5. Verwendung der 9,10-Bis(1,3-dithiol-2-yliden)-9,10-dihydroanthracenpolymere gemäß Anspruch 1 oder 2 als Elektrodenslurry für elektrische Ladungsspeicher, insbesondere Sekundärbatterien.

## Claims

1. 9,10-Bis(1,3-dithiol-2-ylidene)-9,10-dihydroanthracene polymers consisting of an oligomeric or polymeric compound of the general formula I 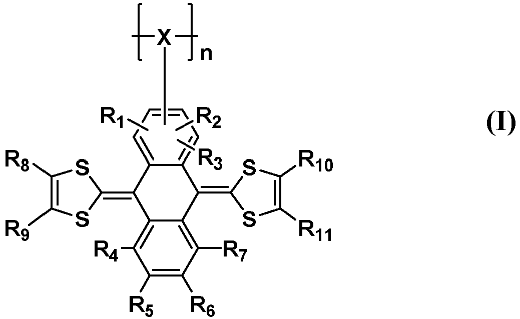 where
R₁ to R₇: are each independently hydrogen atoms, alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, carboxylic acid groups, sulphonic acid groups, amino groups, monoalkylamino groups, dialkylamino groups, nitro groups, cyano groups, hydroxyl groups, alkylcarbonyl groups, alkenylcarbonyl groups, alkynylcarbonyl groups, carboxylic ester groups, carboxamide groups, sulphonic ester groups, thiol groups, halogen atoms or a combination of these groups or atoms, particular preference being given to hydrogen atoms as at least five of the R₁ to R₇ substituents and to non-hydrogen atoms, preferably halogen atoms, alkyl groups, alkoxy groups, cyano groups and/or nitro groups, as zero to two of the R₁ to R₇ substituents,
R₈ to R₁₁: are each independently hydrogen atoms, alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, amino groups, monoalkylamino groups, dialkylamino groups, nitro groups, cyano groups, hydroxyl groups, alkylcarbonyl groups, alkenylcarbonyl groups, alkynylcarbonyl groups, carboxylic ester groups, carboxamide groups, sulphonic ester groups, thiol groups, halogen atoms or a combination of these groups or atoms, where the R₈ and R₉ substituents or the R₁₀ and R₁₁ substituents may form a further ring consisting of five to seven atoms (the ring may be aromatic, heteroaromatic or nonaromatic; if the ring is nonaromatic, it may consist of various groups, for example alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, amino groups, monoalkylamino groups, dialkylamino groups, alkylcarbonyl groups, alkenylcarbonyl groups, alkynylcarbonyl groups, carboxylic ester groups, carboxamide groups, sulphonic ester groups; more preferably, R₈ to R₁₁ are the same and are alkyl groups, such as typically methyl groups or ethyl groups, alkylthio groups, such as methylthio groups, or ethylthio groups and thiol groups),
X: is an organic group which is formed by polymerization reaction from a group consisting of an organic double bond, an organic triple bond, an oxirane or an aziridine, or is an organic group which is formed by a polymer-analogous reaction,
n: is an integer greater than or equal to 2.

2. 9,10-Bis(1,3-dithiol-2-ylidene)-9,10-dihydroanthracene polymers according to Claim 1, **characterized in that** X is an organic group of one of the general formulae II-XIV: 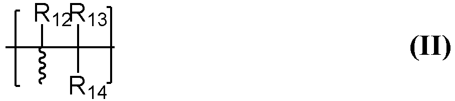 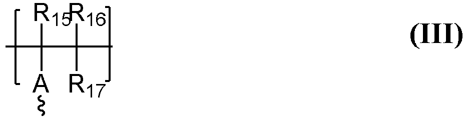 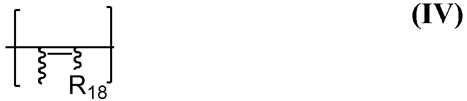 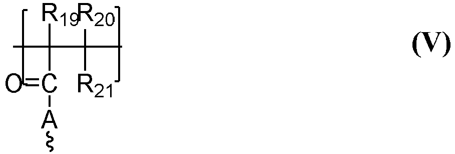 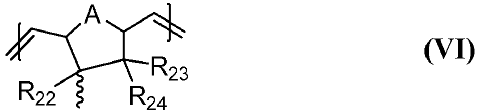 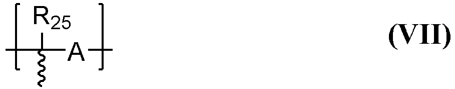 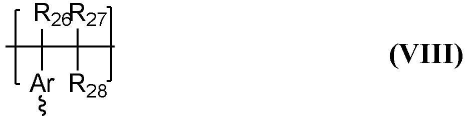 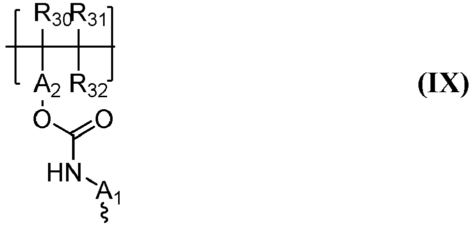 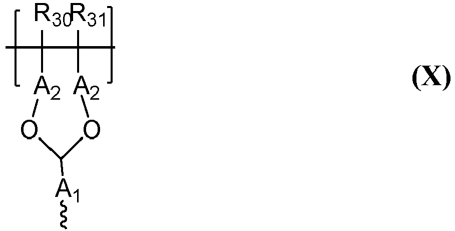 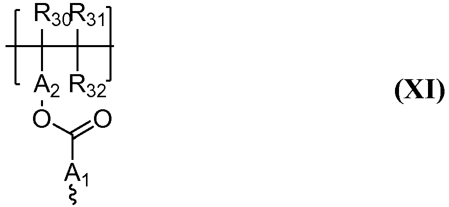 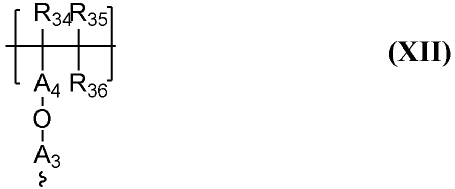 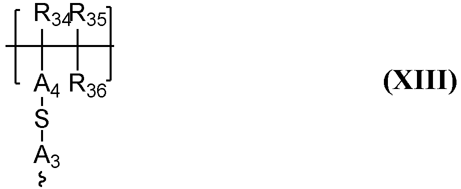 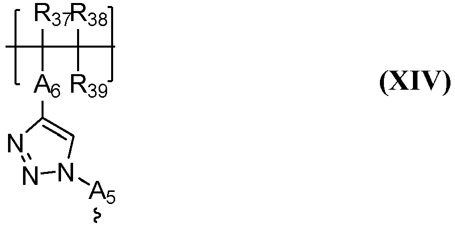 where
R₁₂ to R₂₈: are each independently hydrogen atoms, alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, carboxylic acid groups, sulphonic acid groups, amino groups, monoalkylamino groups, dialkylamino groups, nitro groups, cyano groups, hydroxyl groups, alkylcarbonyl groups, alkenylcarbonyl groups, alkynylcarbonyl groups, carboxylic ester groups, carboxamide groups, sulphonic ester groups, thiol groups, halogen atoms or a combination of these groups or atoms, particular preference being given to hydrogen atoms as at least two of the R₁₂ to R₁₄ substituents and to non-hydrogen atoms, preferably halogen atoms, alkyl groups, alkoxy groups, cyano groups and/or nitro groups, as zero to two of the R₁₂ to R₁₄ substituents, and/or
hydrogen atoms as at least two of the R₁₅ to R₁₇ substituents and to non-hydrogen atoms, preferably halogen atoms, alkyl groups, alkoxy groups, cyano groups and/or nitro groups, as zero to one of the R₁₅ to R₁₇ substituents, and/or
a hydrogen atom as R₁₈, and/or
hydrogen atoms as at least two of the R₁₉ to R₂₁ substituents and to non-hydrogen atoms, preferably halogen atoms, alkyl groups, alkoxy groups, cyano groups and/or nitro groups, as zero to one of the R₁₉ to R₂₁ substituents, and/or
hydrogen atoms as at least two of the R₂₂ to R₂₄ substituents and to non-hydrogen atoms, preferably halogen atoms, alkyl groups, alkoxy groups, cyano groups and/or nitro groups, as zero to one of the R₂₁₂ to R₂₄ substituents, and/or
a hydrogen atom as R₂₅, and/or
hydrogen atoms as at least two of the R₂₆ to R₂₈ substituents and to non-hydrogen atoms, preferably halogen atoms, alkyl groups, alkoxy groups, cyano groups and/or nitro groups, as zero to one of the R₂₆ to R₂₈ substituents,
R₃₀ to R₃₂: are each independently hydrogen atoms, alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, carboxylic acid groups, sulphonic acid groups, amino groups, monoalkylamino groups, dialkylamino groups, nitro groups, cyano groups, hydroxyl groups, alkylcarbonyl groups, alkenylcarbonyl groups, alkynylcarbonyl groups, carboxylic ester groups, carboxamide groups, sulphonic ester groups, thiol groups, halogen atoms or a combination of these groups or atoms, particular preference being given to hydrogen atoms as at least two of the R₃₀ to R₃₂ substituents and to non-hydrogen atoms, preferably halogen atoms, alkyl groups, alkoxy groups, cyano groups and/or nitro groups, as zero to one of the R₃₀ to R₃₂ substituents,
R₃₄ to R₃₆: are each independently hydrogen atoms, alkyl groups, alkenyl groups, alkynyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, carboxylic acid groups, sulphonic acid groups, amino groups, monoalkylamino groups, dialkylamino groups, nitro groups, cyano groups, alkylcarbonyl groups, alkenylcarbonyl groups, alkynylcarbonyl groups, carboxylic ester groups, carboxamide groups, sulphonic ester groups, halogen atoms or a combination of these groups or atoms, particular preference being given to hydrogen atoms as at least two of the R₃₄ to R₃₆ substituents and to non-hydrogen atoms, preferably halogen atoms, alkyl groups, alkoxy groups, cyano groups and/or nitro groups, as zero to one of the R₃₄ to R₃₆ substituents,
R₃₇ to R₃₉: are each independently hydrogen atoms, alkyl groups, alkenyl groups, alkoxy groups, alkylthio groups, haloalkyl groups, haloalkoxy groups, cycloalkyl groups, cycloalkoxy groups, aryl groups, heteroaryl groups, aryloxy groups, aralkyl groups, carboxylic acid groups, sulphonic acid groups, amino groups, monoalkylamino groups, dialkylamino groups, nitro groups, cyano groups, hydroxyl groups, alkylcarbonyl groups, alkenylcarbonyl groups, alkynylcarbonyl groups, carboxylic ester groups, carboxamide groups, sulphonic ester groups, thiol groups, halogen atoms or a combination of these groups or atoms, particular preference being given to hydrogen atoms as at least two of the R₃₇ to R₃₉ substituents and to non-hydrogen atoms, preferably halogen atoms, alkyl groups, alkoxy groups, cyano groups and/or nitro groups, as zero to one of the R₃₇ to R₃₉ substituents,
A: is an oxygen atom, a sulphur atom or an-N(R₃₃)- group, where R₃₃ is a hydrogen atom, an alkyl group, an alkenyl group, an alkynyl group, an alkoxy group, an alkylthio group, a haloalkyl group, a haloalkoxy group, a cycloalkyl group, a cycloalkoxy group, an aryl group, a heteroaryl group, an aryloxy group, an aralkyl group, a carboxylic acid group, a sulphonic acid group, a nitro group, an alkylcarbonyl group, an alkenylcarbonyl group, an alkynylcarbonyl group, a carboxylic ester group, a carboxamide group, a sulphonic ester group, particular preference being given to an oxygen atom as A,
A₁ and A₂: are each independently a covalent bond, an alkyl group, an alkenyl group, an alkynyl group, an alkoxy group, an alkylthio group, a haloalkyl group, a haloalkoxy group, a cycloalkyl group, a cycloalkoxy group, an aryl group, a heteroaryl group, an aryloxy group, an aralkyl group, a monoalkylamino group, a dialkylamino group, an alkylcarbonyl group, an alkenylcarbonyl group, an alkynylcarbonyl group, a carboxylic ester group, a carboxamide group, a sulphonic ester group, particular preference being given to a covalent bond or an alkyl group as A₁ and A₂,
A₃ and A₄: are each independently a covalent bond, an alkyl group, an alkenyl group, an alkynyl group, an alkoxy group, an alkylthio group, a cycloalkyl group, a cycloalkoxy group, an aryl group, a heteroaryl group, an aryloxy group, an aralkyl group, a dialkylamino group, an alkylcarbonyl group, an alkenylcarbonyl group, an alkynylcarbonyl group, a carboxylic ester group, a carboxamide group, a sulphonic ester group, particular preference being given to a covalent bond or an alkyl group as A₁ and A₂,
A₅ and A₆: are each independently a covalent bond, an alkyl group, an alkenyl group, an alkoxy group, an alkylthio group, a haloalkyl group, a haloalkoxy group, a cycloalkyl group, a cycloalkoxy group, an aryl group, a heteroaryl group, an aryloxy group, an aralkyl group, a monoalkylamino group, a dialkylamino group, an alkylcarbonyl group, an alkenylcarbonyl group, an alkynylcarbonyl group, a carboxylic ester group, a carboxamide group, a sulphonic ester group, particular preference being given to a covalent bond, an aryl group or an alkyl group as A₅ and A₆,
Ar: is an independently substituted cycloalkyl group, cycloalkoxy group, aryl group, heteroaryl group, aryloxy group, aralkyl group.

3. Use of the 9,10-bis(1,3-dithiol-2-ylidene)-9,10-dihydroanthracene polymers according to Claim 1 or 2 as active electrode material for electrical charge storage means, especially secondary batteries.

4. Use of the 9,10-bis(1,3-dithiol-2-ylidene)-9,10-dihydroanthracene polymers according to Claim 3, **characterized in that** the active electrode material takes the form of a full or partial surface coating of electrode elements of the electrical charge storage means, especially secondary batteries.

5. Use of the 9,10-bis(1,3-dithiol-2-ylidene)-9,10-dihydroanthracene polymers according to Claim 1 or 2 as electrode slurry for electrical charge storage means, especially secondary batteries.

## Revendications

1. Polymères de 9,10-bis(1,3-dithiol-2-ylidène)-9,10-dihydroanthracène, constitués par un composé oligomère ou polymère de formule générale I 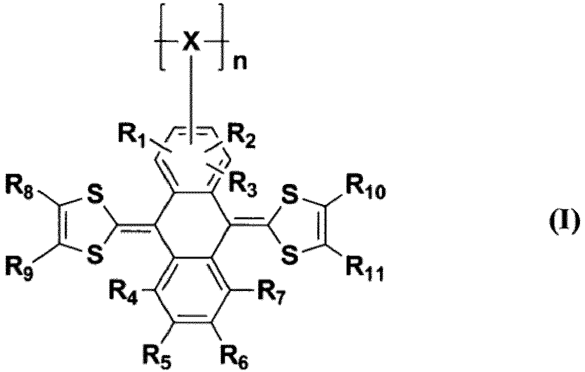 dans laquelle
R₁ à R₇ : représentent indépendamment les uns des autres des atomes d'hydrogène, des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes acide carboxylique, des groupes acide sulfonique, des groupes amino, des groupes monoalkylamino, des groupes dialkylamino, des groupes nitro, des groupes cyano, des groupes hydroxyle, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des groupes thiol, des atomes d'halogène ou une combinaison de ces groupes ou atomes, des atomes d'hydrogène étant particulièrement préférés en tant qu'au moins cinq des substituants R₁ à R₇, et des atomes autres que l'hydrogène, de préférence des atomes d'halogène, des groupes alkyle, des groupes alcoxy, des groupes cyano et/ou des groupes nitro, en tant que zéro à deux des substituants R₁ à R₇,
R₈ à R₁₁: représentent indépendamment les uns ces autres des atomes d'hydrogène, des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes amino, des groupes monoalkylamino, des groupes dialkylamino, des groupes nitro, des groupes cyano, des groupes hydroxyle, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des groupes thiol, des atomes d'halogène ou une combinaison de ces groupes ou atomes, les substituants R₈ et R₉ et respectivement les substituants R₁₀ et R₁₁ pouvant former un cycle supplémentaire, qui est constitué par cinq à sept atomes (le cycle peut être aromatique, hétéroaromatique ou non aromatique ; si le cycle est non aromatique, il peut être constitué par différents groupes, tels que par exemple des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes amino, des groupes monoalkylamino, des groupes dialkylamino, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, de manière particulièrement préférée, R₈ à R₁₁ se correspondent et sont des groupes alkyle, tels que par exemple des groupes méthyle ou des groupes éthyle, des groupes alkylthio, tels que des groupes méthylthio, ou des groupes éthylthio et des groupes thiol),
X : est un groupe organique, qui est formé par une réaction de polymérisation à partir d'un groupe qui est constitué par une double liaison organique, une triple liaison organique, un oxirane ou une aziridine, ou est un groupe organique, qui est formé par une réaction polymère-analogue,
n est un nombre entier supérieur ou égal à 2.

2. Polymères de 9,10-bis(1,3-dithiol-2-ylidène)-9,10-dihydroanthracène selon la revendication 1, **caractérisés en ce que** X représente un groupe organique d'une des formules générales II à XIV 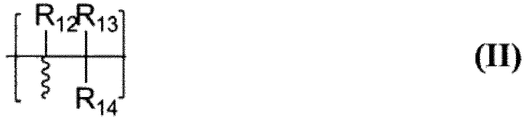 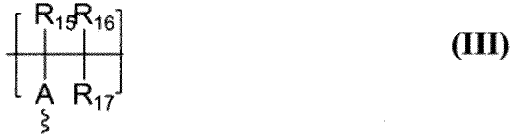 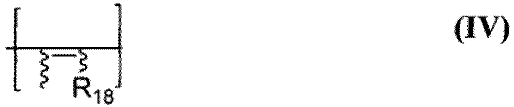 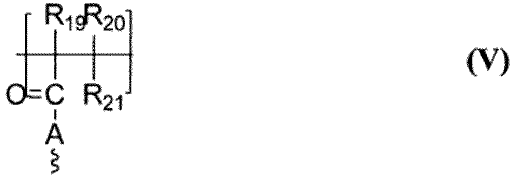 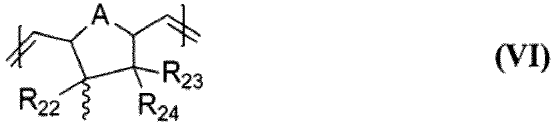 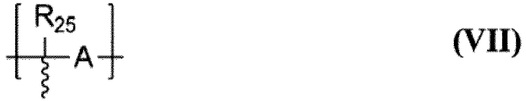 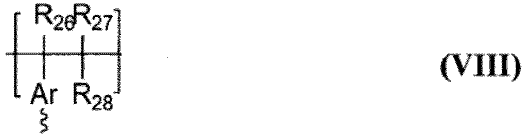 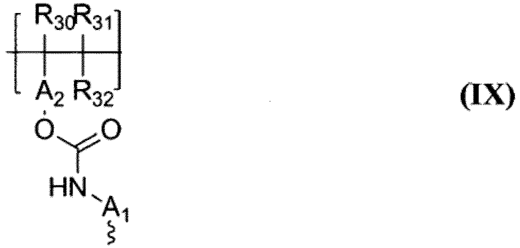 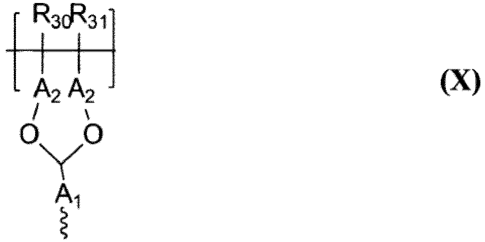 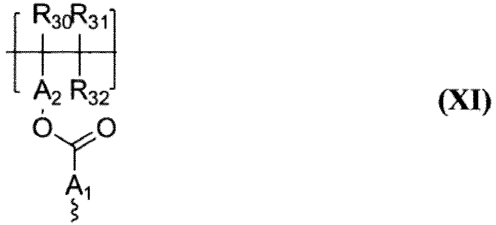 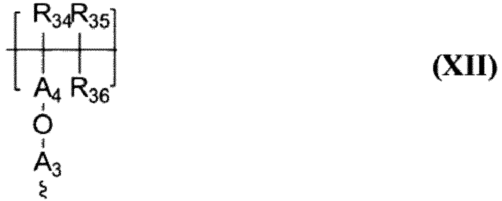 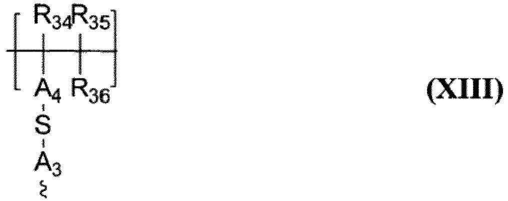 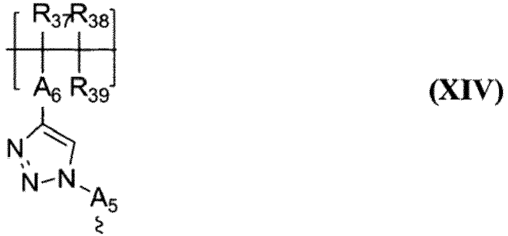 dans lesquelles
R₁₂ à R₂₈ : représentent indépendamment les uns des autres des atomes d'hydrogène, des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes acide carboxylique, des groupes acide sulfonique, des groupes amino, des groupes monoalkylamino, des groupes dialkylamino, des groupes nitro, des groupes cyano, des groupes hydroxyle, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des groupes thiol, des atomes d'halogène ou une combinaison de ces groupes ou atomes,
des atomes d'hydrogène étant particulièrement préférés en tant qu'au moins deux des substituants R₁₂ à R₁₄, et des atomes autres que l'hydrogène, de préférence des atomes d'halogène, des groupes alkyle, des groupes alcoxy, des groupes cyano et/ou des groupes nitro, en tant que zéro à deux des substituants R₁₂ à R₁₄, et/ou des atomes d'hydrogène étant particulièrement préférés en tant qu'au moins deux des substituants R₁₅ à R₁₇, et des atomes autres que l'hydrogène, de préférence des atomes d'halogène, des groupes alkyle, des groupes alcoxy, des groupes cyano et/ou des groupes nitro, en tant que zéro à un des substituants R₁₅ à R₁₇, et/ou un atome d'hydrogène étant particulièrement préféré en tant que R₁₈, et/ou
des atomes d'hydrogène étant particulièrement préférés en tant qu'au moins deux des substituants R₁₉ à R₂₁, et des atomes autres que l'hydrogène, de préférence des atomes d'halogène, des groupes alkyle, des groupes alcoxy, des groupes cyano et/ou des groupes nitro, en tant que zéro à un des substituants R₁₉ à R₂₁, et/ou
des atomes d'hydrogène étant particulièrement préférés en tant qu'au moins deux des substituants R₂₂ à R₂₄, et des atomes autres que l'hydrogène, de préférence des atomes d'halogène, des groupes alkyle, des groupes alcoxy, des groupes cyano et/ou des groupes nitro, en tant que zéro à un des substituants R₂₁ à R₂₄, et/ou un atome d'hydrogène étant particulièrement préféré en tant que R₂₅, et/ou
des atomes d'hydrogène étant particulièrement préférés en tant qu'au moins deux des substituants R₂₆ à R₂₈, et des atomes autres que l'hydrogène, de préférence des atomes d'halogène, des groupes alkyle, des groupes alcoxy, des groupes cyano et/ou
des groupes nitro, en tant que zéro à un des substituants R₂₆ à R₂₈,
R₃₀ à R₃₂: représentent indépendamment les uns ces autres des atomes d'hydrogène, des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes acide carboxylique, des groupes acide sulfonique, des groupes amino, des groupes monoalkylamino, des groupes dialkylamino, des groupes nitro, des groupes cyano, des groupes hydroxyle, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des groupes thiol, des atomes d'halogène ou une combinaison de ces groupes ou atomes, des atomes d'hydrogène étant particulièrement préférés en tant qu'au moins deux des substituants R₃₀ à R₃₂, et des atomes autres que l'hydrogène, de préférence des atomes d'halogène, des groupes alkyle, des groupes alcoxy, des groupes cyano et/ou des groupes nitro, en tant que zéro à un des substituants R₃₀ à R₃₂,
R₃₄ à R₃₆: représentent indépendamment les uns des autres des atomes d'hydrogène, des groupes alkyle, des groupes alcényle, des groupes alcynyle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes acide carboxylique, des groupes acide sulfonique, des groupes amino, des groupes monoalkylamino, des groupes dialkylamino, des groupes nitro, des groupes cyano, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des atomes d'halogène ou une combinaison de ces groupes ou atomes, des atomes d'hydrogène étant particulièrement préférés en tant qu'au moins deux des substituants R₃₄ à R₃₆, et des atomes autres que l'hydrogène, de préférence des atomes d'halogène, des groupes alkyle, des groupes alcoxy, des groupes cyano et/ou des groupes nitro, en tant que zéro à un des substituants R₃₄ à R₃₆,
R₃₇ à R₃₉ : représentent indépendamment les uns des autres des atomes d'hydrogène, des groupes alkyle, des groupes alcényle, des groupes alcoxy, des groupes alkylthio, des groupes haloalkyle, des groupes haloalcoxy, des groupes cycloalkyle, des groupes cycloalcoxy, des groupes aryle, des groupes hétéroaryle, des groupes aryloxy, des groupes aralkyle, des groupes acide carboxylique, des groupes acide sulfonique, des groupes amino, des groupes monoalkylamino, des groupes dialkylamino, des groupes nitro, des groupes cyano, des groupes hydroxyle, des groupes alkylcarbonyle, des groupes alcénylcarbonyle, des groupes alcynylcarbonyle, des groupes ester d'acide carboxylique, des groupes amide d'acide carboxylique, des groupes ester d'acide sulfonique, des groupes thiol, des atomes d'halogène ou une combinaison de ces groupes ou atomes, des atomes d'hydrogène étant particulièrement préférés en tant qu'au moins deux des substituants R₃₇ à R₃₉, et des atomes autres que l'hydrogène, de préférence des atomes d'halogène, des groupes alkyle, des groupes alcoxy, des groupes cyano et/ou des groupes nitro, en tant que zéro à un des substituants R₃₇ à R₃₉,
A : représente un atome d'oxygène, un atome de soufre ou un groupe -N(R₃₃)-, R₃₃ représentant un atome d'hydrogène, un groupe alkyle, un groupe alcényle, un groupe alcynyle, un groupe alcoxy, un groupe alkylthio, un groupe haloalkyle, un groupe haloalcoxy, un groupe cycloalkyle, un groupe cycloalcoxy, un groupe aryle, un groupe hétéroaryle, un groupe aryloxy, un groupe aralkyle, un groupe acide carboxylique, un groupe acide sulfonique, un groupe nitro, un groupe alkylcarbonyle, un groupe alcénylcarbonyle, un groupe alcynylcarbonyle, un groupe ester d'acide carboxylique, un groupe amide d'acide carboxylique, un groupe ester d'acide sulfonique ; un atome d'oxygène étant particulièrement préféré en tant qu'A,
A₁ et A₂ : représentent indépendamment l'un de l'autre une liaison covalente, un groupe alkyle, un groupe alcényle, un groupe alcynyle, un groupe alcoxy, un groupe alkylthio, un groupe haloalkyle, un groupe haloalcoxy, un groupe cycloalkyle, un groupe cycloalcoxy, un groupe aryle, un groupe hétéroaryle, un groupe aryloxy, un groupe aralkyle, un groupe monoalkylamino, un groupe dialkylamino, un groupe alkylcarbonyle, un groupe alcénylcarbonyle, un groupe alcynylcarbonyle, un groupe ester d'acide carboxylique, un groupe amide d'acide carboxylique, un groupe ester d'acide sulfonique ; une liaison covalente ou un groupe alkyle étant particulièrement préféré en tant qu'A₁ et A₂,
A₃ et A₄ : représentent indépendamment l'un de l'autre une liaison covalente, un groupe alkyle, un groupe alcényle, un groupe alcynyle, un groupe alcoxy, un groupe alkylthio, un groupe cycloalkyle, un groupe cycloalcoxy, un groupe aryle, un groupe hétéroaryle, un groupe aryloxy, un groupe aralkyle, un groupe dialkylamino, un groupe alkylcarbonyle, un groupe alcénylcarbonyle, un groupe alcynylcarbonyle, un groupe ester d'acide carboxylique, un groupe amide d'acide carboxylique, un groupe ester d'acide sulfonique ; une liaison covalente ou un groupe alkyle étant particulièrement préféré en tant qu'A₁ et A₂,
A₅ et A₆ : représentent indépendamment l'un de l'autre une liaison covalente, un groupe alkyle, un groupe alcényle, un groupe alcoxy, un groupe alkylthio, un groupe haloalkyle, un groupe haloalcoxy, un groupe cycloalkyle, un groupe cycloalcoxy, un groupe aryle, un groupe hétéroaryle, un groupe aryloxy, un groupe aralkyle, un groupe monoalkylamino, un groupe dialkylamino, un groupe alkylcarbonyle, un groupe alcénylcarbonyle, un groupe alcynylcarbonyle, un groupe ester d'acide carboxylique, un groupe amide d'acide carboxylique, un groupe ester d'acide sulfonique ; une liaison covalente, un groupe aryle ou un groupe alkyle étant particulièrement préféré en tant qu'A₅ et A₆,
Ar : représente un groupe cycloalkyle, un groupe cycloalcoxy, un groupe aryle, un groupe hétéroaryle, un groupe aryloxy, un groupe aralkyle, substitués indépendamment les uns des autres.

3. Utilisation des copolymères de 9,10-bis(1,3-dithiol-2-ylidène)-9,10-dihydroanthracène selon la revendication 1 ou 2 en tant que matériau d'électrode actif pour des accumulateurs de charge électriques, notamment des batteries secondaires.

4. Utilisation des copolymères de 9,10-bis(1,3-dithiol-2-ylidène)-9,10-dihydroanthracène selon la revendication 3, **caractérisée en ce que** le matériau d'électrode actif est configuré sous la forme d'un revêtement de surface complet ou partiel d'éléments d'électrodes des accumulateurs de charge électriques, notamment des batteries secondaires.

5. Utilisation des copolymères de 9,10-bis(1,3-dithiol-2-ylidène)-9,10-dihydroanthracène selon la revendication 1 ou 2 en tant que pâte d'électrode pour des accumulateurs de charge électriques, notamment des batteries secondaires.
